# EUROPEAN PATENT SPECIFICATION

(11) **EP 4 343 778 B1**
(45) Date of publication and mention of the grant of the patent: **18.02.2026**
(21) Application number: 23821853.1
(22) Date of filing: 27.07.2023
(51) Int. Cl.: G16H 30/20, G06T 7/00, G06T 5/90, G06T 11/00

(54) **IMAGE PROCESSING METHOD, APPARATUS AND SYSTEM, AND STORAGE MEDIUM**
BILDGEBUNGSVERARBEITUNGSVERFAHREN, VORRICHTUNGEN, SYSTEME UND SPEICHERMEDIEN DAFÜR
PROCÉDÉ, APPAREIL ET SYSTÈME DE TRAITEMENT D'IMAGE, ET SUPPORT DE STOCKAGE

(30) Priority: 27.07.2022 CN 202210893798; 12.06.2023 CN 202310691687; 12.06.2023 CN 202310692397
(43) Date of publication of application: 27.03.2024
(73) Proprietor: Shanghai United Imaging Healthcare Co., Ltd., Shanghai 201807 (CN)
(72) Inventor: LI, Jingjing, Shanghai 201807 (CN); MA, Runxia, Shanghai 201807 (CN); LYU, Yang, Shanghai 201807 (CN); JI, Zijun, Shanghai 201807 (CN); LIU, Shitao, Shanghai 201807 (CN); WANG, Jianxun, Shanghai 201807 (CN); WANG, Kang, Shanghai 201807 (CN)
(74) Representative: Wang, Bo
(86) International application number: PCT/CN2023/109694
(87) International publication number: WO 2024/022461

(56) References cited:
- CN-A- 110 013 263
- CN-A- 113 409 220
- CN-A- 115 206 499
- CN-A- 115 206 499
- US-A1- 2010 290 680
- US-A1- 2014 126 794
- US-A1- 2015 356 754
- US-A1- 2020 029 918
- US-A1- 2020 258 271
- KAALEP ANDRES ET AL: "Quantitative implications of the updated EARL 2019 PET-CT performance standards", vol. 6, no. 1, 1 December 2019 (2019-12-01), XP093200625, ISSN: 2197-7364, Retrieved from the Internet <URL:https://ejnmmiphys.springeropen.com/counter/pdf/10.1186/s40658-019-0257-8.pdf> [retrieved on 20240902], DOI: 10.1186/s40658-019-0257-8

## Description

### CROSS-REFERENCE TO RELATED APPLICATIONS

This application claims priority to Chinese Application No. 202210893798.1, filed on July 27, 2022, Chinese Application No. 202310691687.7, filed on June 12, 2023, and Chinese Application No. 202310692397.4, filed on June 12, 2023.

### TECHNICAL FIELD

The present disclosure relates to the field of medical image processing technique, and in particular, to a PET image processing method, system, and storage medium thereof.

### BACKGROUND

A standard uptake value (SUV) is a semi-quantitative index commonly used in the diagnosis of tumors by positron emission computed tomography (PET). The SUV is extensively utilized for identifying benign and malignant tumors, as well as evaluating therapeutic efficacy and predicting prognosis. Generally, the more malignant the lesion is, the higher the SUV may be. Due to the differences in body weight and body surface area of different subjects during the examination, as well as differences in physiological factors of the subjects, examination processes, etc., the accuracy and repeatability of the SUV are affected. Therefore, the normalization of the SUV may be needed to standardize and control the factors influencing the SUV and eliminate the quantitative differences caused by body size and injected dose to some extent. However, in practical application, for reasons such as the device model or reconstruction algorithms, etc., some PET/PET-CT devices may not be able to normalize the recovery coefficient (RC) curve of the phantom image at this time to the range specified by EARL using a post-processing algorithm. This leads to the inability to compare the SUVs of PET images acquired under reconstruction conditions across devices, patients, and time, thus failing to meet the clinical needs. Besides, the current SUV normalization methods primarily rely on providing an input box at the workstation for physicians to enter the corresponding filter parameters. Subsequently, the loaded images are processed, and the SUV is recalculated. This approach is not only time-consuming but also lacks user-friendliness.

Accordingly, it is desired to provide a PET image processing method, system, device, and storage medium to improve the efficiency and accuracy of medical image processing, to achieve SUV comparability across devices, patients, and time, and to meet clinical needs. Relevant prior art is disclosed in "Quantitative implications of the updated EARL 2019 PET-CT performance standards", by Kaalep, A., Burggraaff, C.N., Pieplenbosch, S. et al., EJNMMI Phys 6, 28 (2019), https://doi.org/10.1186/ s40658-019-0257-8, which aims at investigating the impact of the EARL2 specifications on the quantitative reads of clinical PET-CT studies, and CN115206499A, which relates to an image processing method.

### SUMMARY

The invention is defined by the independent claims, the dependent claims relate to embodiments.

### BRIEF DESCRIPTION OF THE DRAWINGS

The present disclosure is further illustrated in terms of exemplary embodiments. These exemplary embodiments are described in detail according to the drawings. These embodiments are non-limiting exemplary embodiments, in which like reference numerals represent similar structures, wherein:
FIG. 1 is a schematic diagram illustrating an application scenario of an image processing system according to some embodiments of the present disclosure;
FIG. 2 is a diagram illustrating exemplary modules of an image processing system according to some embodiments of the present disclosure;
FIG. 3 is a flowchart illustrating an exemplary process for image processing according to some embodiments of the present disclosure;
FIG. 4 is a flowchart illustrating an exemplary process for illustrating obtaining a target processing parameter according to some embodiments of the present disclosure;
FIG. 5A is a schematic diagram illustrating a working interface and a visualization interface of an image processing system according to some embodiments of the present disclosure;
FIG. 5B is a schematic diagram illustrating a working interface and a visualization interface of an image processing system according to some embodiments of the present disclosure;
FIG. 6 is a flowchart illustrating an exemplary process for determining a normalized standard uptake value according to some embodiments of the present disclosure;
FIG. 7 is a schematic diagram illustrating an upper bound, a lower bound, an expected value of RCₘₐₓ in a reference standard, and a first recovery coefficient corresponding to RCₘₐₓ satisfying the reference standard according to some embodiments of the present disclosure;
FIG. 8 is a flowchart illustrating an exemplary process for determining a customized standard according to some embodiments of the present disclosure;
FIG. 9 is a flowchart illustrating an exemplary process for determining a normalized standard uptake value according to some embodiment of the present disclosure;
FIG. 10 is a schematic diagram illustrating a semi-quantitative analysis based on a normalized standard uptake value according to some embodiments of the present disclosure;
FIG. 11 is a flowchart illustrating an exemplary process for determining a normalized standard uptake value according to some embodiments of the present disclosure;
FIG. 12 is an exemplary flowchart for determining a normalization factor based on an EARL V1.0 standard according to some embodiments of the present disclosure;
FIG. 13 is a flowchart illustrating an exemplary process for determining a normalization factor based on EARL V2.0 according to some embodiments of the present disclosure;
FIG. 14 is a schematic diagram illustrating a structure of a computer that may realize all or part of the functions of a processing device 140 of an image processing system according to some embodiments of the present disclosure;
FIG. 15A is a schematic diagram illustrating a phantom according to some embodiments of the present disclosure; and
FIG. 15B is a schematic diagram illustrating an exemplary phantom image according to some embodiments of the present disclosure.

### DETAILED DESCRIPTION

To more clearly illustrate the technical solutions related to the embodiments of the present disclosure, a brief introduction of the drawings referred to the description of the embodiments is provided below. Obviously, the drawings described below are only some examples or embodiments of the present disclosure. Those having ordinary skills in the art, without further creative efforts, may apply the present disclosure to other similar scenarios according to these drawings.

It should be understood that "system", "device", "unit" and/or "module" as used herein is a manner used to distinguish different components, elements, parts, sections, or assemblies at different levels. However, if other words serve the same purpose, the words may be replaced by other expressions.

As shown in the present disclosure and claims, the words "one", "a", "a kind" and/or "the" are not especially singular but may include the plural unless the context expressly suggests otherwise. In general, the terms "comprise," "comprises," "comprising," "include," "includes," and/or "including," merely prompt to include operations and elements that have been clearly identified, and these operations and elements do not constitute an exclusive listing. The methods or devices may also include other operations or elements.

The flowcharts used in the present disclosure illustrate operations that systems implement according to some embodiments of the present disclosure. It should be understood that the previous or subsequent operations may not be accurately implemented in order. Instead, each step may be processed in reverse order or simultaneously. Meanwhile, other operations may also be added to these processes, or a certain step or several steps may be removed from these processes.

Due to the differences in body weight and body surface area of different subjects, as well as differences in physiological factors of the subjects, examination processes, etc., accuracy and repeatability of a normalized standard uptake value (SUV) are affected. There is therefore a need to normalize the SUV, standardize and control factors influencing the SUV, and to some extent eliminate quantitative differences caused by body size and injected dose. The current SUV normalization methods primarily rely on providing an input box at the workstation for physicians to enter the corresponding filter parameters, and then loaded images are processed and the SUV is recalculated. However, there lack of an automated method to obtain the filter parameters, and for different images, the required filter parameters need to be manually calculated by doctors. This manual process is time-consuming and not userfriendly, and the calculation standard may not be selected. In addition, the filter parameters calculated each time need to be transferred to the workstation through paper slips and thus cannot be saved.

Some embodiments of the present disclosure provide an image processing method, device, system, and storage medium, which calculate filter parameters based on an acquisition parameter and a reconstruction parameter of a phantom image to form a set of data, which is recorded in a configuration file of the system. The next time the image is processed, the acquisition parameter and the reconstruction parameter are read and matched with the data in the configuration file, and if the matching is successful, the image is directly normalized using matched filter parameters. Thus, phantom data from different manufacturers is analyzed, and the analysis process has a progress prompt as well as an intuitive graph, which is easy for a user to watch. It flexibly selects a standard (such as an EARL V1.0 standard and an EARL V2.0 standard) for automatic calculation, without the need for doctors to manually calculate, which improves efficiency. In addition, a filter parameter value is also automatically saved and transferred, replacing the use of small notes, making it convenient for later review and analysis.

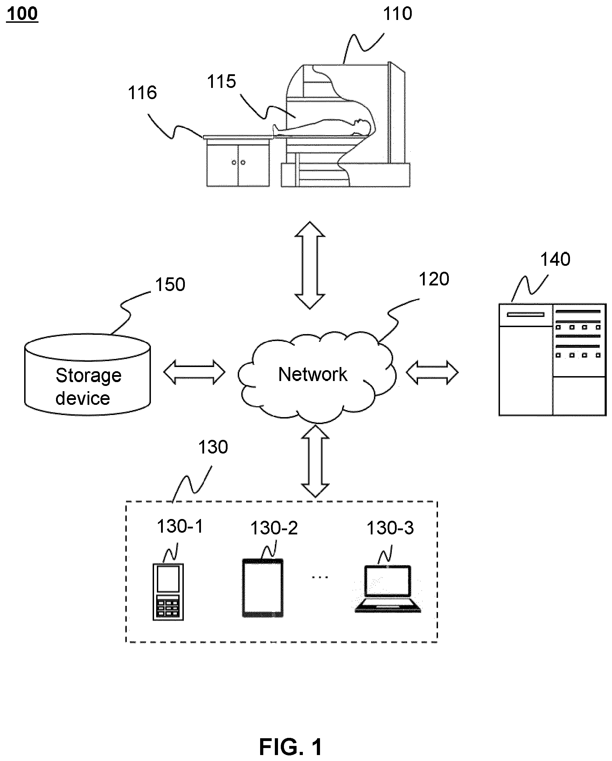

FIG. 1 is a schematic diagram illustrating an application scenario of an image processing system according to some embodiments of the present disclosure.

As shown in FIG. 1, an application scenario 100 of an image processing system (hereinafter referred to as the application scenario 100) may include a medical imaging device 110, a network 120, a terminal device 130, a processing device 140, and a storage device 150. Components in the application scenario 100 may be connected in a plurality of ways. Merely by way of example, as shown in FIG. 1, the medical imaging device 110 may be connected with the processing device 140 via the network 120. The storage device 150 may be connected with the processing device 140 either directly or via the network 120.

The medical imaging device 110 may scan a scanned subject and/or generate data about the scanned subject. Exemplarily, the medical imaging device 110 may include a PET device, a PET-CT device, etc. In some embodiments, the scanned subject may also be referred to as a scanned object, a target object, a target, or a detected object. In some embodiments, the target object may be a patient, an animal, etc. In some embodiments, the target object may be a phantom, etc. The target object may enter a scanning region 115 through an examination bed 116, and the medical imaging device 110 may scan the target object to obtain a medical image (e.g., a phantom image, an image to be processed, etc.) corresponding to the target object.

The network 120 may include any suitable network that facilitates the exchange of information and/or data for application scenario 100. In some embodiments, one or more components of the application scenario 100 (e.g., the medical imaging device 110, the terminal device 130, the processing device 140, and the storage device 150) may be connected via the network 120 to transmit information and/or data with one or more other components of the application scenario 100. For example, the processing device 140 may obtain a phantom image, etc., from the medical imaging device 110 via the network 120. In some embodiments, the network 120 may be any one or more of a wired network or a wireless network. In some embodiments, the network may be any of a plurality of topologies, such as peer-to-peer, shared, centralized, or a combination of topologies.

The terminal device 130 may include a mobile device 130-1, a tablet 130-2, a laptop 130-3, etc., or any combination thereof. In some embodiments, the terminal device 130 may be integrated with the medical imaging device 110. In some embodiments, the terminal device 130 may interact with other components in the application scenario 100 via the network 120. For example, the terminal device 130 may receive data such as the phantom image, the image to be processed, etc. sent by the medical imaging device 110. In some embodiments, the terminal device 130 may receive information and/or an instruction input by a user (e.g., a user of the medical imaging device 110, such as a doctor) and, via the network 120, send the received information and/or instruction to the medical imaging device 110 or the processing device 140. For example, a doctor may input an operation instruction for the medical imaging device 110 via the terminal device 130. In some embodiments, the terminal device 130 may display the phantom image, the image to be processed, an image processing result, etc. In some embodiments, the terminal device 130 may also be integrated with the medical imaging device 110.

In some embodiments, the terminal device 130 may include a display interface for displaying information related to the medical image. For example, the phantom image, the image to be processed, the image processing result, a reference processing parameter, and visualization data generated by the reference matching parameter, etc. may be displayed according to the display interface.

The processing device 140 may process data and/or information obtained from the medical imaging device 110, the terminal device 130, and/or the storage device 150. For example, the processing device 140 may obtain the phantom image and a normalization standard; perform a first processing on the phantom image, generate a recovery coefficient of the phantom image after the first processing, and determine whether the recovery coefficient satisfies the normalization standard; in response to determining that the recovery coefficient satisfies the normalization standard, obtain a target processing parameter, wherein the target processing parameter is a processing parameter of the first processing when the recovery coefficient satisfies the normalization standard; determine an image processing result by processing the image to be processed based on the target processing parameter.

In some embodiments, the processing device 140 may be a single server or a server group. The server group may be centralized or distributed. In some embodiments, the processing device 140 may be local or remote. The processing device 140 may be directly connected with the medical imaging device 110, the terminal device 130, and the storage device 150 to access stored information or obtained information and/or data. In some embodiments, the processing device 140 may be implemented on a cloud platform. Merely by way of example, the cloud platform may include a private cloud, a public cloud, a hybrid cloud, a community cloud, a distributed cloud, an on-premises cloud, a multi-tier cloud, etc., or any combination thereof.

The storage device 150 may store data and/or instructions. In some embodiments, the storage device 150 may store data obtained from the medical imaging device 110, the terminal device 130, and/or the processing device 140. For example, the storage device 150 may store a phantom image obtained by scanning a phantom. In some embodiments, the storage device may also be configured to store an image processing parameter, an image processing result, etc. In some embodiments, the storage device 150 may be configured to store data and/or instructions used by the processing device 140 to perform the exemplary method described in the present disclosure. In some embodiments, the storage device 150 may include a mass storage device, a removable storage device, volatile randomaccess memory, read-only memory (ROM), etc., or any combination thereof. In some embodiments, the storage device 150 may be implemented on a cloud platform.

In some embodiments, the storage device 150 may be connected with the network 120 to communicate with one or more components of the application scenario 100 (e.g., the medical imaging device 110, the terminal device 130, the processing device 140, etc.). One or more components of the application scenario 100 may access data or instructions stored in the storage device 150 via the network 120. In some embodiments, the storage device 150 may be directly connected with or communicate with one or more components of the application scenario 100. In some embodiments, the storage device 150 may be part of the processing device 140.

It should be noted that the application scenario 100 is provided for illustrative purposes only and is not intended to limit the scope of the present disclosure. Those skilled in the art may make various changes and modifications to the present disclosure according to the description of the present disclosure. For example, the terminal device 130, and the processing device 140 may share a common storage device or have their own storage devices. However, these changes and modifications are still within the scope of the present disclosure.

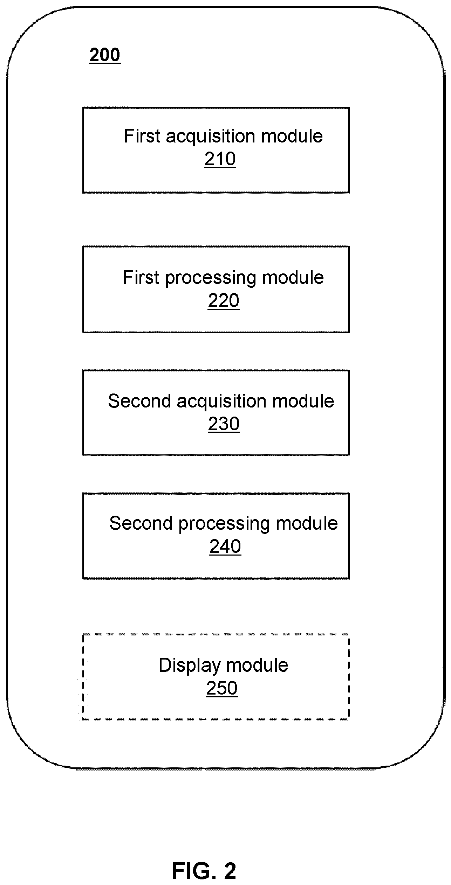

FIG. 2 is a diagram illustrating exemplary modules of an image processing system according to some embodiments of the present disclosure. As shown in FIG. 2, an image processing system 200 (hereinafter referred to as the system 200) includes a first acquisition module 210, a first processing module 220, a second acquisition module 230, and a second processing module 240.

The first acquisition module 210 is configured to obtain a phantom image and a normalization standard.

The first processing module 220 is configured to perform a first processing on the phantom image, generate a recovery coefficient of the phantom image by performing the first processing on the phantom image, and determine whether the recovery coefficient satisfies a normalization standard.

The second acquisition module 230 is configured to obtain a target processing parameter in response to determining that the recovery coefficient satisfies the normalization standard. The target processing parameter is a processing parameter of the first processing when the recovery coefficient satisfies the normalization standard.

In some embodiments, different phantom images are obtained based on different reference matching parameters. The second acquisition module 230 is further configured to obtain a reference matching parameter and a reference processing parameter corresponding to the phantom image and generate a preset configuration file, wherein the reference matching parameter and the reference processing parameter are stored associatively in a preset configuration file; obtain a parameter to be matched corresponding to the image to be processed based on an image to be processed; match the parameter to be matched with the reference matching parameter in the preset configuration file; and in response to determining that the matching between the parameter to be matched and a reference matching parameter is successful, obtain the reference processing parameter associated with the reference matching parameter as the target processing parameter.

In some embodiments, each of the parameter to be matched and the reference matching parameter includes an acquisition parameter and a reconstruction parameter.

The second processing module 240 is configured to perform a second processing on the image to be processed based on a target processing parameter to determine an image processing result.

In some embodiments, the normalization standard includes a reference standard and a customized standard, and the image processing result includes a normalized standard uptake value. The second processing module 240 is further configured to obtain the phantom image and the reference standard; perform the first processing on the phantom image to obtain a first recovery coefficient, in response to determining that the first recovery coefficient does not satisfy the reference standard, determine the customized standard as a target normalization standard; obtain a second recovery coefficient by performing a plurality of iterations, and in each iteration, perform the first processing on the phantom image, in response to determining that the second recovery coefficient satisfies the customized standard ,obtain the target processing parameter; perform a second processing on the image to be processed based on the target processing parameter, and determine the normalized standard uptake value, wherein the target processing parameter is a processing parameter when the second recovery coefficient satisfies the customized standard.

In some embodiments, the customized standard may be determined based on obtaining a user input parameter.

In some embodiments, the user input parameter may be determined based on a value of the first recovery coefficient corresponding to a sphere in a phantom.

In some embodiments, the customized standard includes a maximum recovery coefficient, a mean recovery coefficient, and a regional mean value of a peak recovery coefficient. Each of the maximum recovery coefficient, the mean recovery coefficient, and the regional mean value of the peak recovery coefficient includes an expected value, an upper bound, and a lower bound, respectively. The second processing module 240 is further configured to determine the expected value based on the value of the first recovery coefficient corresponding to the sphere.

In some embodiments, the reference standard includes at least one of an EARL V1.0 standard and an EARL V2.0 standard.

In some embodiments, the phantom image and the image to be processed are obtained based on the same acquisition condition and reconstruction condition.

In some embodiments, the normalized standard includes a reference standard. The image processing result includes the normalized standard uptake value. The second processing module 240 is further configured to determine the target processing parameter based on the phantom image and the reference standard, wherein the target processing parameter is a normalization factor; perform a second processing on the image to be processed using the normalization factor to determine the normalized standard uptake value.

In some embodiments, the reference standard includes an EARL V1.0 standard. The normalization factor includes at least one of a first factor, a second factor, and a third factor. The second processing module 240 is further configured to perform a plurality of iterations, and in each iteration, perform the first processing on the phantom image, and determine the first factor and the second factor based on an expected value of a standard parameter in EARL V1.0 standard, wherein the standard parameter in the EARL V1.0 standard includes a maximum recovery factor and an average recovery factor; determine the third factor by a first preset manner based on the first factor and said second factor.

In some embodiments, the first preset manner includes determining the third factor based on a maximum value of the first factor and a maximum value of the second factor.

In some embodiments, the reference standard includes an EARL V2.0 standard, and the normalization factor includes at least one of the first factor, the second factor, and the third factor. The second processing module 240 is further configured to calculate, by a second preset manner, an expected value of the standard parameter based on an upper bound, and a lower bound of the standard parameter in the EARL V2.0 standard, wherein the standard parameter in the EARL V2.0 standard includes at least one of a maximum recovery coefficient, a mean recovery coefficient and a regional mean value of a peak recovery coefficient; perform a plurality of iterations, and in each iteration, perform the first processing on the phantom image, and determine at least one of the first factor, the second factor, and the third factor based on the expected value of the standard parameter.

In some embodiments, the second preset manner includes determining the expected value of the standard parameter based on an average value of the upper bound and the lower bound of the standard parameter.

In some embodiments, the second processing module 240 is further configured to determine a normalized image by performing the second processing on the image to be processed based on the target processing parameter; determine a normalized quantitative result based on the normalized image and generate an image processing result based on the normalized quantitative result.

In some embodiments, the system 200 may also include a display module 250.

In some embodiments, the display module 250 is configured to generate visualization data based on the reference processing parameter and the reference matching parameter.

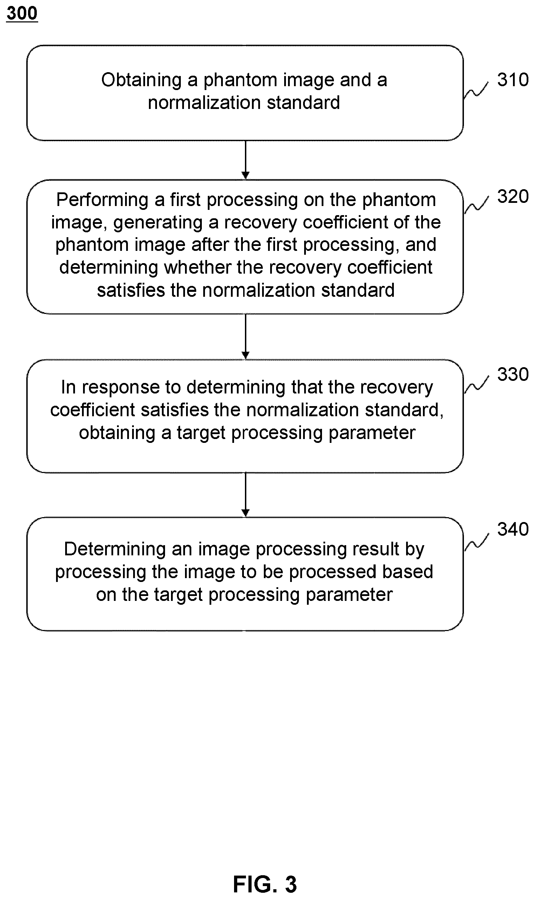

More descriptions regarding the normalization standard, the recovery coefficient, the reference matching parameter, the reference processing parameter, etc., may be found in descriptions of other parts of the present disclosure (e.g., FIG. 3, etc.).

It should be noted that the above description of the image processing system 200 and modules thereof is provided only for descriptive convenience and does not limit the present disclosure to the scope of the cited embodiments. It is to be understood that for those skilled in the art, after understanding the principle of the system, it may be possible to arbitrarily combine each of the modules or form a subsystem to connect with other modules without departing from the principle. In some embodiments, the first acquisition module 210, the first processing module 220, the second acquisition module 230, the second processing module 240, and the display module 250 disclosed in FIG. 2 may be different modules in a system or a single module that implements functions of two or more of the above-described modules. For example, each of the modules may share a common storage module, and each of the modules may have its storage module. Such deformations are within the protection scope of the present disclosure.

FIG. 3 is a flowchart illustrating an exemplary process for image processing according to some embodiments of the present disclosure. As shown in FIG. 3, process 300 includes the following operations. In some embodiments, the process 300 may be performed by the processing device 140 or the system 200.

In 310, a phantom image and a normalization standard are obtained.

The phantom image refers to an image obtained by image acquisition and image reconstruction based on infusing a radioactive substance into a phantom. The image acquisition refers to obtaining an aggregation situation of the radioactive substance in the phantom, and the image reconstruction refers to imaging (e.g., positron emission computed tomography, i.e., PET) based on the aggregation situation of the radioactive substance. The phantom includes a plurality of spheres, the plurality of spheres may represent organs, tissues, etc., of a target object (e.g., a human body), and the volume of the plurality of spheres may be different. The phantom including six spheres is taken as an example in the following description.

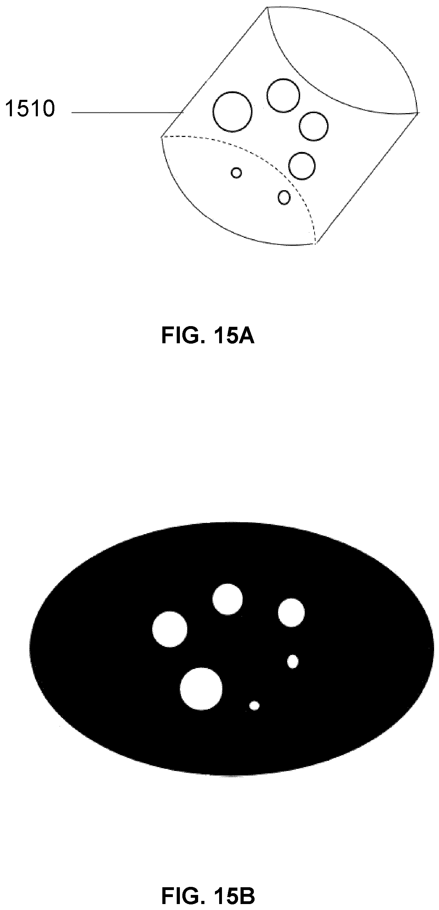

FIG. 15A is a schematic diagram illustrating a phantom according to some embodiments of the present disclosure. FIG. 15B is a schematic diagram illustrating an exemplary phantom image according to some embodiments of the present disclosure. As shown in FIG. 15A, the phantom 1510 includes six spheres, and a phantom image is obtained by infusing a radioactive substance into the phantom and performing image acquisition on the phantom. In some embodiments, when the phantom is infused with the radioactive substance, the background region in the phantom and the six spheres are infused at a concentration of 1:10. The phantom image is shown in FIG. 15B, where highlighted portions indicate the six spheres and the remaining portion indicates the background region. The phantom 1510 may be a NEMA IQ phantom, which is a physical phantom that is designed and produced based on a National Electrical Manufacturers Association (NEMA) standard, and primarily used for standardized verification of the performance of an Emission Computed Tomography (ECT) system for nuclear medicine.

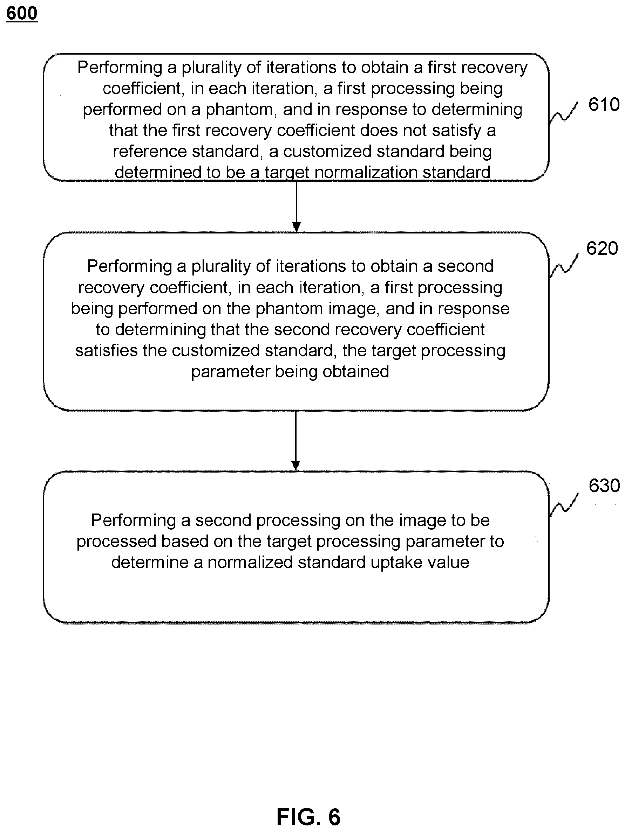

The normalization standard is a standard that normalizes a standard uptake value. More descriptions regarding the normalization standard may be found in FIG. 6 and related descriptions thereof.

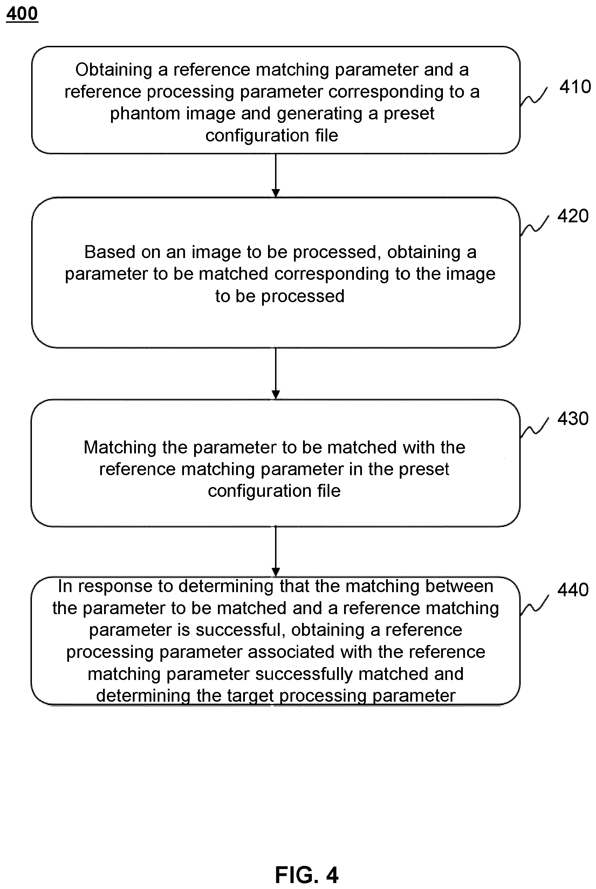

The phantom image may be obtained by the processing device based on image acquisition and image reconstruction of the phantom by the medical imaging device 110. For example, the processing device may obtain the phantom image by performing image acquisition and image reconstruction on the phantom based on a preset parameter (e.g., a reference matching parameter). More details regarding the reference matching parameter may be found in FIG. 4 and related descriptions thereof.

In some embodiments, the processing device may also obtain an image to be processed based on image acquisition and image reconstruction on the target object to be processed (e.g., a patient or animal, etc.) by the medical imaging device. The image to be processed refers to an image that needs to be processed (e.g., by performing SUV normalization) for medical analysis (e.g., SUV semi-quantitative analysis). For example, the image to be processed may include an image of the human body that need to be subjected to SUV semi-quantitative analysis. The process of SUV normalization on the image to be processed may also be referred to as a second processing. In some embodiments, the image to be processed may be a PET image.

In 320, a first processing is performed on the phantom image, a recovery coefficient of the phantom image after the first processing is generated, and whether the recovery coefficient satisfies the normalization standard is determined.

The first processing refers to a processing of performing an image post-processing (e.g., Gaussian filtering) on the phantom image. Exemplarily, the first processing may include performing at least one iteration on the phantom by f processing a recovery coefficient (RC) curve of the phantom image via Gaussian filtering, wherein each iteration of the at least one iteration has different filter parameters. A count of the iterations may be determined based on a preset.

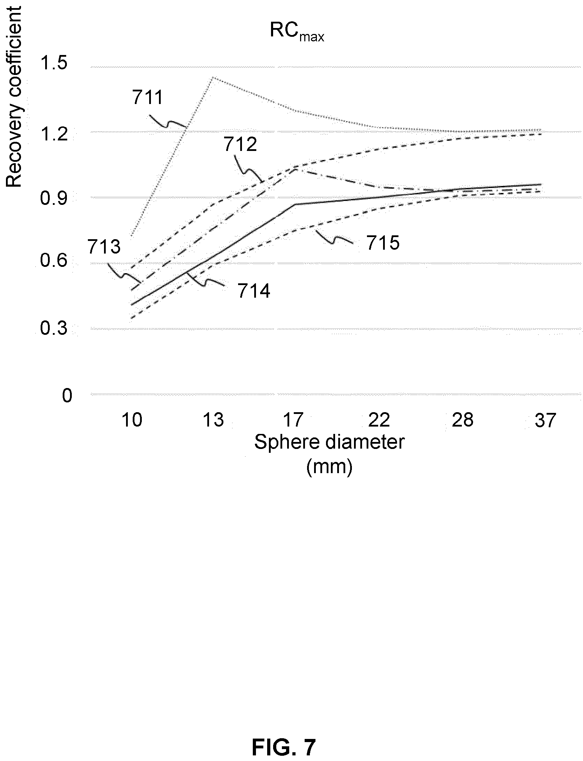

The recovery coefficient refers to a ratio of the activity ratio of the radioactive substance of one of the plurality of spheres actually monitored after the radioactive substance is infused into the plurality of spheres to the activity ratio of the infused radioactive substance. For example, when the phantom is infused with the radioactive substance, the infusion concentration of the six spheres is 10, the infusion concentration of the background region is 1, and an activity ratio is 10/1=10. Whereas the infusion concentration of one of the six spheres that is actually monitored is 9, the infusion concentration of the background region is 0.95, the activity ratio is about 9.47, and the recovery coefficient of the sphere is 9.47/10=0.947. The recovery coefficient may be expressed by one or more RC curves. The RC curves may include curves consisting of the recovery coefficients of spheres under three different definitions, including an RCₘₐₓ curve, an RCₘₑₐₙ curve, and an RCₚₑₐₖ curve. More descriptions regarding the RC curves may be found in FIG. 6, FIG. 7, etc., and related descriptions thereof.

In some embodiments, the processing device may process a RC curve of the phantom image by performing Gaussian filtering for a plurality of iterations to generate the recovery coefficient of the phantom image after the first processing. A condition for terminating the plurality of iterations may include that a count of the plurality of iterations reach a maximum count or the generated recovery coefficient satisfies a reference standard.

More descriptions regarding determining whether the recovery coefficient satisfies the normalization standard may be found in relevant portions hereinafter of the present disclosure, such as FIG. 6 and FIG. 7.

In 330, in response to determining that the recovery coefficient satisfies the normalization standard, a target processing parameter is obtained.

The target processing parameter is a parameter that is ultimately used to perform normalization (the second processing) on the image to be processed.

In some embodiments, the processing device may determine the target processing parameter based on the recovery coefficient and the normalization standard. Exemplarily, the processing device may perform the first processing on the RC curve of the phantom image for the plurality of iterations and record the filter parameter used for each of the plurality of iterations, determine whether each of a plurality of recovery coefficients corresponding to the plurality of iterations of the first processing satisfies the normalization standard, record a filter parameter when the normalization standard is satisfied, and determine the filter parameter as the target processing parameter.

In some embodiments, the processing device may obtain the target processing parameter from a preset configuration file based on matching a reference matching parameter corresponding to the phantom image and a reference matching parameter of the image to be processed. More related descriptions may be found in FIG. 4.

In 340, an image processing result is determined by processing the image to be processed based on the target processing parameter.

The image processing result refers to data generated after performing a second processing on the image to be processed. For example, the image processing result includes a normalized quantitative result, a clinically derived indicator corresponding to the normalized image, etc.

In some embodiments, the processing device may determine the image processing result by performing the second processing on the image to be processed based on the target processing parameter. For example, the processing device may determine the image processing result by performing Gaussian filtering on the image to be processed based on the filter parameter in the target processing parameter.

In some embodiments, the processing device may also determine the normalized image by performing the second processing on the image to be processed based on the target processing parameter and determine the image processing result based on the normalized image, and the image processing result includes the normalized quantitative result.

The normalized image refers to the image to be processed after the second processing (normalization).

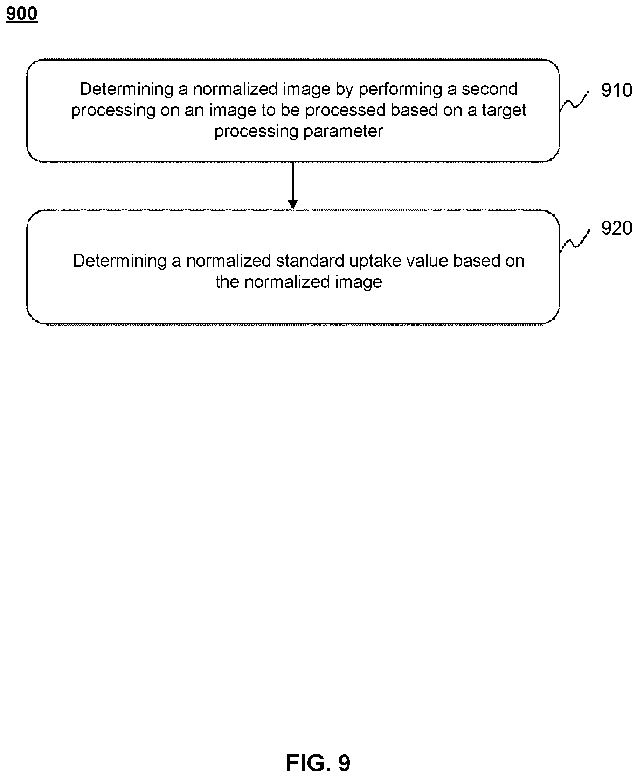

The normalized quantitative result refers to a normalized standardized uptake value, which may include u.SUVₘₑₐₙ, u.SUVₘₐₓ, and u.SUVₚₑₐₖ. More descriptions regarding the normalized standard uptake value may be found in FIG. 9.

In some embodiments, the processing device may directly determine the normalized quantitative result as the image processing result.

In some embodiments, the normalization standard may include a reference standard and a customized standard, and the image processing result may further include the normalized standard uptake value. The processing device may determine, the normalized standard uptake value based on the first recovery coefficient, the second recovery coefficient, and the target processing parameter. More related descriptions may be found in FIG. 6, etc.

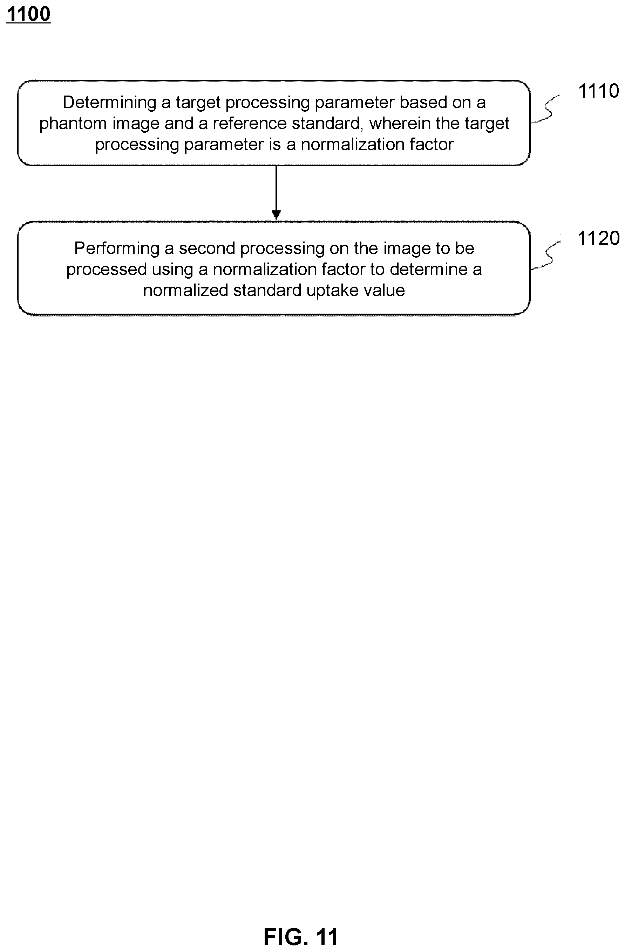

In some embodiments, the normalization standard may include the reference standard, and the image processing result may include the normalized standard uptake value. The processing device may determine the target processing parameter based on the phantom image and the reference standard. The target processing parameter is a normalization factor. The normalization factor is used to perform the second processing on the image to be processed to determine the normalized standard uptake value. More related descriptions may be found in FIG. 11, etc.

The image processing method described in some embodiments of the present disclosure may achieve at least the following effects. (1) Phantom images of different types may be analyzed, with a wide range of applications. (2) The normalization standard may be flexibly selected, which eliminates the need for the tedious operation of a doctor to manually calculate the processing parameter, thereby improving the accuracy and efficiency of the normalization process.

In some embodiments, different phantom images may be obtained based on different reference matching parameters, and the manner of obtaining the target processing parameter may be implemented based on process 400.

FIG. 4 is a flowchart illustrating an exemplary process for obtaining a target processing parameter according to some embodiments of the present disclosure. As shown in FIG. 4, process 400 includes the following operations. In some embodiments, the process 400 may be performed by the processing device 140 or the system 200.

In 410, a reference matching parameter and a reference processing parameter corresponding to a phantom image are obtained and a preset configuration file is generated.

The reference matching parameter refers to a parameter used for obtaining the phantom image based on the acquisition and reconstruction.

The reference processing parameter refers to a parameter used for performing a first processing on the phantom image to make a recovery coefficient of the phantom image after the first processing satisfy a normalization standard. The reference processing parameter may be a filter parameter (e.g., including a size of a Gaussian filter, etc.).

In some embodiments, each of a parameter to be matched and the reference matching parameter includes an acquisition parameter and a reconstruction parameter. More details regarding the parameter to be matched may be found in the related descriptions in operation 420.

The acquisition parameter may include but is not limited to an image acquisition duration, a concentration of a radioactive substance infused before the acquisition, a scanning speed, an acquisition mode, a decay correction, etc., for obtaining the phantom image and the image to be processed.

The reconstruction parameter may include but is not limited to, an image reconstruction algorithm, a matrix size, a layer thickness, a count of iterations, etc.

The reference matching parameter may be obtained based on historical phantom image acquisition and reconstruction process and stored in the storage device 150.

The preset configuration file refers to a pre-generated file used for matching to obtain a target processing parameter, and the preset configuration file may include a reference matching parameter and a reference processing parameter. The reference matching parameter and the reference processing parameter of the same phantom image are stored associatively in the preset configuration file.

In some embodiments, the processing device may generate the preset configuration file based on the reference matching parameter and the reference processing parameter of the phantom image.

In some embodiments, the processing device may determine the reference processing parameter using a manner similar to the manner of obtaining the target processing parameter in operations 310 to 330. That is, the first processing is performed on the phantom image in a plurality of iterations, and the filter parameter when the recovery coefficient satisfies the normalization standard after the first processing is determined as the reference processing parameter.

In some embodiments, the normalization standard may include a reference standard and a customized standard. The reference standard is a common normalization standard of a standard uptake value, i.e., an EARL standard. The EARL standard refers to a range obtained based on a NEMA IQ phantom database, and the EARL standard may include a standard range that needs to be satisfied by RC curves of 6 spheres in a phantom after being normalized, i.e., an upper bound, a lower bound, and an expected value.

The EARL standard may include an EARL V1.0 standard and an EARL V2.0 standard. Manners of analyzing data for the phantom image for the EARL V1.0 standard and the EARL V2.0 standard are different. For example, an operator may choose to analyze the phantom image via the EARL V1.0 standard and/or the EARL V2.0 standard by an intelligent image processing software tool, taking into account the actual situation. By filtering the recovery coefficient of the image that satisfies the above-described ERAL standard, and based on a filter parameter corresponding to the recovery coefficient that satisfies a filtering condition, the reference processing parameter corresponding to the phantom image may be obtained by calculation.

More descriptions regarding the customized standard may be found elsewhere in the present disclosure, e.g., in FIG. 6.

In some embodiments, the processing device may also obtain a corresponding recovery coefficient by calculating the phantom image after the first processing; compare the recovery coefficient with a standard parameter range of the normalization standard to obtain the reference processing parameter.

In response to a comparison result indicating that the recovery coefficient is within the standard parameter range, a full width at half maxima of a Gaussian function corresponding to the recovery coefficient is obtained, and based on the full width at half maxima, the reference processing parameter is generated by calculation.

In response to the comparison result indicating that the recovery coefficient is outside the standard parameter range, the full width at half maxima is adjusted, a new recovery coefficient based on the adjusted full width at half maxima is correspondingly obtained until the new recovery coefficient is within the standard parameter range, and the reference processing parameter is obtained based on the adjusted full width at half maxima.

The full width at half maxima of the Gaussian function refers to a quantitative metric used to characterize a spatial extent caused by a partial volume effect, which may also be referred to as a filter parameter. Exemplarily, the processing device may obtain a plurality of image recovery coefficients by an exhaustive method, etc. For example, any six transverse layers may be selected in the phantom image, at each thermal foci of each transverse layer, a circular region of interest with size the same as the real transverse size of the thermal foci is sketched out, and a corresponding image recovery coefficient is calculated based on a radioactivity concentration in each region of interest. Exemplarily, the processing device may also design full widths at half maxima of a plurality of Gaussian functions by the exhaustive method, etc. For example, the processing device may calculate the full widths at half maxima corresponding to the six hot foci described above. It may be understood that the full width at half maxima of each of the Gaussian functions is generally within a range of 0 mm-10 mm, and there is generally an interval of 0.1 mm between the full widths at half maxima of adjacent Gaussian functions.

After obtaining two standard filter lines through data analysis based on the normalization standard, the processing device may compare the recovery coefficient of each phantom image with the standard parameter range indicated by the two standard filter lines, to obtain the reference processing parameter.

If a recovery coefficient located between the two standard filter lines is matched, or a recovery coefficient that coincides with or has a high repetition degree with the standard filter lines is matched, the phantom image is considered to conform to a PET image standard. Therefore, a post-processing adjustment may not be performed on the phantom image, and a standard filter may be generated directly based on the full width at half maxima of the Gaussian function corresponding to the recovery coefficient. A size of the standard filter is the reference processing parameter.

If the detected image recovery coefficient is not within the standard parameter range, the phantom image is considered not to conform to the PET image standard, and the full widths at half maxima may be re-adjusted to generate a new recovery coefficient by calculation. The new recovery coefficient may be compared again with the standard parameter range of the normalization standard until a recovery coefficient within the standard parameter range is detected, and the reference processing parameter is generated.

In some embodiments, the processing device may obtain the reference matching parameter based on the acquisition parameter and the reconstruction parameter when obtaining and reconstructing the phantom image and generate the preset configuration file based on the reference processing parameter and the reference matching parameter.

In some embodiments, after the processing device obtains the reference processing parameter of the phantom image by calculation, the reference processing parameter, as well as a parameter such as the reference matching parameter (the acquisition parameter and the reconstruction parameter) used to generate the phantom image, are further integrated and saved in the preset configuration file. The preset configuration file may be stored in the storage device 150.

In some embodiments, the preset configuration file may include a plurality of groups of reference matching parameters and a plurality of groups of reference processing parameters corresponding to a plurality of phantom images, each of the plurality of groups of the reference matching parameters and each of the plurality of groups of the reference processing parameters are stored associatively.

It may be understood that the reference processing parameter is obtained by post-processing the phantom image, thus realizing a one-click manner of generating the reference processing parameter without the need for the operator to manually carry out calculations, which is conducive to improving the accuracy and efficiency of image processing.

In 420, based on an image to be processed, a parameter to be matched corresponding to the image to be processed is obtained.

The parameter to be matched refers to a parameter used for obtaining the image to be processed. The parameter to be matched may include an acquisition parameter and a reconstruction parameter.

In some embodiments, the processing device may obtain, based on the image to be processed, the parameter to be matched corresponding to the image to be processed. For example, the processing device may obtain the acquisition parameter and the reconstruction parameter when obtaining and reconstructing the image to be processed and determine the acquisition parameter and the reconstruction parameter as the parameter to be matched. More descriptions regarding the parameter to be matched may be found in relevant portions hereinabove, e.g., operation 310.

In some embodiments, the processing device may obtain adjustment information of a region of interest and adjustment information of an activity ratio of a user for the image to be processed and preprocess the image to be processed based on the adjustment information of the region of interest and the adjustment information of the activity ratio to obtain a pre-processed image and a parameter to be matched corresponding to the pre-processed image.

In some embodiments, the user may interact with the intelligent image processing software tool and adjust parameters such as a region of interest, an activity ratio, etc., that are required to process the image to be processed.

Specifically, in the process of processing the image to be processed, the user may, by clicking on a corresponding button on the intelligent image processing software tool, cause the image processing software tool to generate interaction information such as the adjustment information of the region of interest and the adjustment information of the activity ratio, and the intelligent image processing software tool sends the interaction information to the processing device for processing.

In some embodiments, the user may also directly input corresponding parameter information such as the region of interest, the activity ratio, etc., on the intelligent image processing software tool. Interaction information generated based on user-input information may be sent by the intelligent image processing software tool to the processing device.

It is to be understood that information such as the region of interest, the activity ratio, etc., may also be pre-set by the user and stored in the intelligent image processing software tool, which may not be discussed herein.

In some embodiments, after the processing device receives the adjustment information of the region of interest and the adjustment information of the activity ratio, a scanning device may be instructed to scan based on the adjustment information of the activity ratio and obtain the image to be processed. For example, the adjustment information of the activity ratio may have a background region of 5.3 KBq/mL, the activity of a hot bulb of four times the activity of the background region, and a background volume of 9800 mL.

In some embodiments, the processing device may perform an image segmentation process on the image to be processed based on the adjustment information of the region of interest to obtain a corresponding preprocessed medical image. For example, the processing device may outline a boundary of the tumor as the region of interest in the image to be processed and divide the image to be processed to obtain the preprocessed image, as well as one or more configuration parameters such as the acquisition parameter, the reconstruction parameter, etc. In some embodiments, the processing device may determine the acquisition parameter and the reconstruction parameter corresponding to the pre-processed image as the parameter to be matched.

In 430, the parameter to be matched is matched with the reference matching parameter in the preset configuration file.

In some embodiments, the processing device may match the parameter to be matched with the reference matching parameter in the preset configuration file in a plurality of ways. Exemplarily, the processing device may convert the parameter to be matched and the reference matching parameter in the preset configuration file into vectors for representation, and then determine a vector distance between the vector corresponding to the parameter to be matched and a vector corresponding to each of the reference matching parameters in the present configuration file (e.g., Euclidean distances, cosine distances, etc.). If the vector distance between the vector corresponding to the parameter to be matched and the vector corresponding to each of the reference matching parameters in the present configuration file is less than a preset distance threshold, the matching is successful.

In some embodiments of the present disclosure, the user may adjust parameters such as the region of interest, the activity ratio, etc., through the intelligent image processing software tool, thereby further improving a human-computer interaction between the user and the intelligent image processing software tool, and effectively improving the efficiency of image processing.

In 440, in response to determining that the matching between the parameter to be matched and a reference matching parameter is successful, a reference processing parameter associated with the reference matching parameter successfully matched is obtained and determined as the target processing parameter.

When the matching between the parameter to be matched and the reference matching parameter is successful, the processing device may obtain a reference processing parameter in the preset configuration file associated with the reference matching parameter successfully matched the parameter to be matched and determine the reference processing parameter as the target processing parameter. Further, the processing device may also automatically load the target processing parameter into the image to be processed, process the image to be processed based on the target processing parameter, and determine an image processing result. More descriptions regarding determining the image processing result may be found in relevant portions hereinafter, such as FIG. 6, etc.

When the matching between the parameter to be matched and the reference matching parameter is unsuccessful, the processing device may change the acquisition parameter and the reconstruction parameter of the phantom image, generate a new phantom image as well as a preset configuration file corresponding to the new phantom image, and perform the matching again until the matching is successful.

In some embodiments, the processing device may also send the image processing result to a terminal device for real-time display. Further, after generating the image processing result, the image processing result may be sent to the processing device for real-time display via a visualization interface.

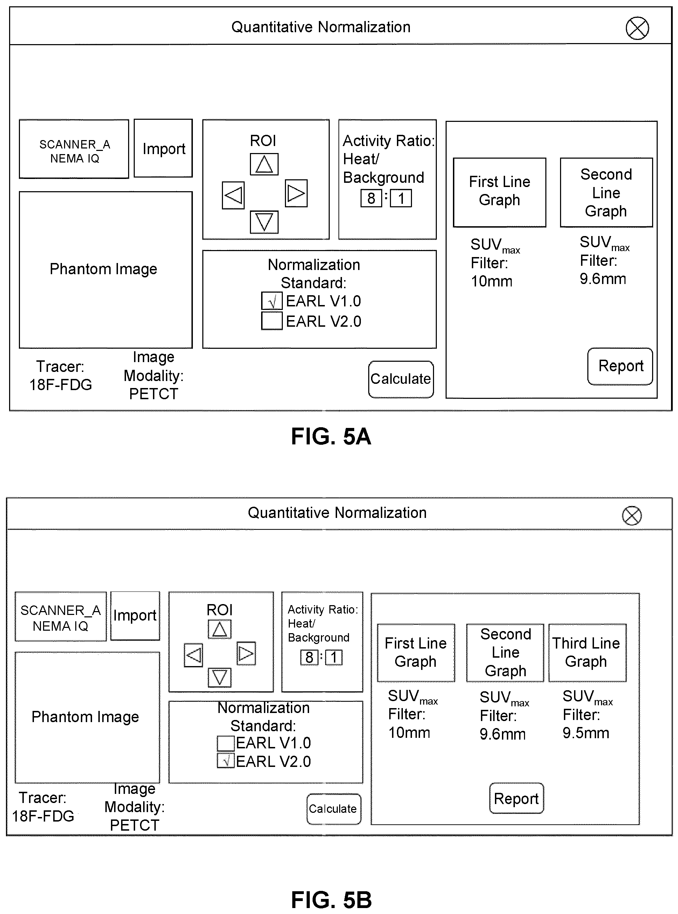

It may be understood that, in an analysis process of the phantom image or the image to be processed, a corresponding analysis progress prompt, a corresponding graph (e.g., a graph of a first recovery coefficient, a graph of a second recovery coefficient, a graph of the normalization standard, etc.), an image processing result (e.g., a normalized SUV), etc., may also be generated independently and displayed to the user by the visualization interface for real-time display. The normalization process of the image to be processed is easier by the automatic loading of the target processing parameter after successful matching. More descriptions regarding the visualization interface may be found in the related descriptions in FIG. 5A and FIG. 5B.

In some embodiments, the processing device may also generate visualization data based on the reference processing parameter and the reference matching parameter. The visualization data may be stored in the terminal device, and the user may input a query instruction (e.g., an instruction for querying the image processing result) via the terminal device, and the terminal device receives the query instruction and displays the visualization data to the user. For example, when the processing device receives query instruction input from the user (e.g., an instruction for querying an RC curve, an instruction for querying a phantom image, etc.), the processing device may display corresponding visualization data to the user.

In some embodiments, the processing device may also generate a visualization comparison result based on a comparison result of comparing the recovery coefficient of the phantom image with the normalization standard. The processing device may also generate the visualization data based on at least the visualization comparison result, the reference matching parameter, and the reference processing parameter and send the visualization data to the terminal device for storage. The terminal device displays the visualization data in response to the query instruction when receiving the query instruction.

The visualization comparison result may be a table consisting of each value of the recovery coefficient and each value corresponding to the standard parameter range in the normalization standard, or the visualization comparison result may be a graph such as a line graph or a bar graph, etc. consisting of values of the recovery coefficient and the standard parameter range, which enables a comparison result between the recovery coefficient and the normalization standard to be visualized by a user.

Specifically, to facilitate research by the user, the intelligent image processing software tool may combined with the visualization comparison result, the full width at half maxima of the Gaussian function, information such as measurement time of a corresponding phantom image, etc., to summarize and generate various forms of visualization data such as a PDF format report or Excel table based on the obtained reference matching parameter, , and send the visualization data to the terminal device. Thus, users may access and query at any time, which effectively improves the usage convenience of the user.

In some embodiments, when different normalization standards are adopted as a reference to determine whether the recovery coefficient of the phantom image after the first processing satisfies the normalization standard, the visualization display interface may display standard parameter ranges in different normalization standards, recovery coefficients corresponding to the standard parameter ranges, the target processing parameter, etc. More details may be found in related descriptions in FIG. 5A and FIG. 5B.

FIG. 5A is a schematic diagram illustrating a working interface and a visualization interface of an image processing system according to some embodiments of the present disclosure.

As shown in FIG. 5A, there is a "Quantitative Normalization" prompt at the top of the working interface and an interactive button in the upper right corner that allows the user to close the image processing software. An image in the left-most area of the visualization interface of the working interface is a phantom image, which is an image reconstructed from scanning a standard workpiece by a medical scanning device.

In some embodiments, the phantom image is a DICOM image. It is to be added that a plurality of spheres with different radii that satisfy a data analysis standard may also be indicated on the phantom image displayed in the visualization interface by highlighting, e.g., by red boxes, i.e., a region of interest (referred to as ROI), which are used to simulate heat and cold illnesses in the body. It may be understood that the radioactivity concentration around the spheres is higher than the radioactivity concentration of other regions of the phantom image.

An import path of a local scanned image is displayed right above the phantom image, and the operator may click the "Import" interactive button on the right side to select a desired path. The SCANNER_A NEMA IQ on the left side of the "Import" interaction button indicates that scanner A is configured to obtain the phantom image based on a NEMA IQ phantom. Displayed directly below the phantom image are the text "Tracer: 18F-FDG" and "Image Modality: PETCT", which are used to prompt the user about the type of a tracer used in the scanning process and the type of image modality generated.

A middle area of the visualization interface displays adjustment button modules corresponding to the ROI, an activity ratio, and a normalization standard, respectively.

As for the ROI, its corresponding adjustment key module may be set as adjustment buttons, so that the operator may adjust it through four adjustment buttons deployed under the display text "ROI", namely "up", "down", "left", and "right".

As for the activity ratio, its corresponding adjustment key module may be set as a ratio input box, the operator may input a ratio of a heat sphere to a background that needs to be set, e.g., an activity ratio is 8:1, in the ratio input box which is deployed under the text "Activity Ratio: Heat/Background".

For the normalization standard, its corresponding adjustment key module may be set as checkable boxes, which may be selected by the operator by checking the checkable boxes in front of the EARL V1.0 standard, the EARL V2.0 standard, and/or the customized standard (not shown in the figure). For example, if the EARL V1.0 standard is checked in FIG. 5A, the processor may perform calculations based on the EARL V1.0 standard.

A "Calculate" interaction button is deployed in the lower right corner of the middle area of the visualization interface, which is used to generate a calculation result with one click. The right-most area of the visualization interface displays a line graph (also known as an RC graph) with eigenvalues and a corresponding filter parameter (i.e., a reference processing parameter, also known as a half width at half maxima of a Gaussian function). When calculating based on the EARL V1.0 standard, the eigenvalues correspond to SUVₘₐₓ and SUVₘₑₐₙ. In the rightmost area, the Filter of SUVₘₐₓ is 10mm, which corresponds to a first line graph when the half width at full maxima of the Gaussian function is 10mm, and the Filter of SUVₘₑₐₙ is 9.6mm, which corresponds to a second line graph when the half width at full maxima of the Gaussian function is 9.6mm. A "Report" interactive button is deployed at the bottom right corner of the right area, which may be used to generate a report result in PDF format with one click.

Specifically, when the user clicks on the "Import" interactive button on the right side of the work interface, a drop-down box on the left side displays one or more import paths that may be selected by the user and imports an image to be processed generated by the current scan. In a process of automatically calculating the image to be processed, the operator may select a corresponding parameter by selecting interactive buttons corresponding to the ROI, the activity ratio, and the normalization standard deployed in the display interface and clicking "Calculate" interactive button in the display interface to realize one-click calculation and get an image processing result. A line graph finally obtained by calculation is displayed in the rightmost area of the visualization interface, and the operator clicks the "Report" interactive button in the visualization interface to generate a visualization report with one click.

FIG. 5B is a schematic diagram illustrating a working interface and a visualization interface of an image processing system according to some embodiments of the present disclosure.

As shown in FIG. 5B, the difference between FIG. 5A and FIG. 5B is that FIG. 5A illustrates the display situation of results obtained when calculating based on the EARL V1.0 standard, and FIG. 5B illustrates the display situation of results obtained when calculating based on the EARL V2.0 standard. It may be understood that due to the lack of definition of RCₚₑₐₖ in the EARL V1.0 standard, the final calculation may only obtain eigenvalues SUVₘₐₓ and SUVₘₑₐₙ, accordingly, the visualization interface of FIG. 5A only shows information corresponding to the eigenvalues SUVₘₐₓ and SUVₘₑₐₙ. When calculating based on the EARL V2.0 standard, the EARL V2.0 standard includes data under RCmax, RCₘₑₐₙ, and RC_{peak.} Therefore, as shown in FIG. 5B, the eigenvalues SUVₘₐₓ and SUVₘₑₐₙ and SUVₚₑₐₖ and their corresponding data may be obtained when calculating based on the EARL V2.0 standard. That is, in the rightmost area, the Filter of SUVₘₐₓ is 10mm, which corresponds to a first line graph when a half width at full maxima of a Gaussian function is 10mm, the Filter of SUVₘₑₐₙ is 9.6mm, which corresponds to a second line graph when the half width at full maxima of the Gaussian function is 9.6mm, and the Filter of SUVₚₑₐₖ is 9.5mm, which corresponds to a third line graph when the half width at full maxima of the Gaussian function is 9.5mm.

In some embodiments of the present disclosure, a working interface and the visualization interface may facilitate the user for querying and operation, which improves processing efficiency. In addition, displaying a historical parameter report and viewing/saving a current parameter report to the user may facilitate the user to perform analysis. When different normalization standards are selected as a reference for calculation, SUV eigenvalues corresponding to the selected normalization standards and their related data may be automatically generated and visualized, making image processing more automated and intelligent.

In one or more embodiments of the present disclosure, in the case of processing an image to be processed on a system where a preset configuration file is stored, the system may match a parameter to be matched with a reference matching parameter in the preset configuration file. If the matching is successful, the system may obtain a reference processing parameter associated with a reference matching parameter successfully matched and take the reference processing parameter as a target processing parameter to process the parameter to be processed, realizing automated filter parameter acquisition, simplifying the operation of the doctor, and improving the accuracy and efficiency of SUV normalization. Furthermore, the method may automatically save and transmit an image processing parameter (i.e., the target processing parameter), replacing the traditional manual recording method, which is easy to query and save.

In an actual operation process of SUV normalization, some PET/CT devices, for reasons such as the model or reconstruction algorithms, etc., some PET/PET-CT devices may not be able to normalize an RC curve of the phantom image at this time to the range specified by an EARL (EANM Research Ltd.) standard (including the EARL V1.0 standard and EARL V2.0 standard) by using a post-processing algorithm (e.g., Gaussian filtering). This leads to the inability to compare an SUV of a PET image obtained under a reconstruction condition across devices, patients, and time, thus failing to meet the clinical needs.

Therefore, in some embodiments of the present disclosure, a customized standard is proposed for SUV normalization when a recovery coefficient of the phantom image cannot satisfy a reference standard. In some embodiments, the normalization standard includes a reference standard and a customized standard. In some embodiments, an image processing result may include a normalized standard uptake value.

FIG. 6 is a flowchart illustrating an exemplary process for determining a normalized standard uptake value according to some embodiments of the present disclosure. In some embodiments, the process 600 may be performed by the processing device 140 or the system 200. As shown in FIG. 6, process 600 includes the following operations.

In 610, a plurality of iterations to obtain a first recovery coefficient may be performed, in each iteration, a first processing is performed on a phantom image, and in response to determining that the first recovery coefficient does not satisfy a reference standard, a customized standard is determined to be a target normalization standard.

The first recovery coefficient refers to an RC parameter obtained after iteratively performing the first processing on the phantom image. In some embodiments, the first recovery coefficient may include a maximum activity value (RCₘₐₓ), a mean activity value (RCₘₑₐₙ), and a mean value of a peak activity region (RCₚₑₐₖ). The mean activity value (RCₘₑₐₙ) refers to an average value of radioactivity ratios in a region represented in the phantom image (a region of interest). In some embodiments, the first recovery coefficient may be represented by an RC curve, i.e., a line connecting RC values of six spheres of a phantom obtained after the first processing. Correspondingly, the RC curves corresponding to the first recovery coefficient may include three types corresponding to the RCₘₐₓ, the RCₘₑₐₙ, and the RCₚₑₐₖ, respectively. Exemplarily, the first recovery coefficient corresponding to the RCₘₐₓ may be a curve 713 as shown in FIG. 7. More descriptions regarding the first processing and the RC curves may be found in FIG. 3.

In some embodiments, the processing device may process the RC curves of the phantom image by performing Gaussian filtering for a plurality of iterations to obtain the first recovery coefficient.

In some embodiments, a condition for terminating the iterations may include the count of iterations that have been performed reaching a maximum count of iterations, or the first recovery coefficient satisfying the normalization standard. More descriptions regarding the normalization standard may be found elsewhere in the present disclosure, e.g., FIG. 4.

In some embodiments, when the first recovery coefficient satisfies the reference standard, the processing device may determine the reference standard as the normalization standard.

In some embodiments, when the first recovery coefficient does not satisfy the reference standard, the processing device may determine the customized standard to be the target normalization standard, i.e., the standard that is ultimately used to normalize the phantom image.

In some embodiments, the reference standard may include at least one of an EARL V1.0 standard or an EARL V2.0 standard. The EARL standard may include a standard range, i.e., at least one of an upper bound, a lower bound, and an expected value, that needs to be satisfied by the RC curves of the six spheres in the phantom after normalization. More descriptions regarding the EARL standard may be found in FIG. 4 and related description thereof. Exemplarily, Table 1 shows the RCₘₐₓ and RCₘₑₐₙ corresponding to the six spheres in the EARL V1.0 standard, and corresponding upper bounds, lower bounds, and expected values thereof.

**Table 1**

| Sphere Volume(ml) | RCₘₐₓ | | | RCₘₑₐₙ | | |
|---|---|---|---|---|---|---|
| | Expected RC | Min RC | Max RC | Expected RC | Min RC | Max RC |
| 26.52 | 0.98 | 0.88 | 1.08 | 0.77 | 0.71 | 0.83 |
| 11.49 | 0.95 | 0.85 | 1.05 | 0.73 | 0.67 | 0.79 |
| 5.57 | 0.89 | 0.77 | 1.01 | 0.66 | 0.59 | 0.73 |
| 2.57 | 0.84 | 0.75 | 0.94 | 0.6 | 0.53 | 0.68 |
| 1.15 | 0.63 | 0.51 | 0.74 | 0.45 | 0.38 | 0.52 |
| 0.52 | 0.38 | 0.29 | 0.46 | 0.3 | 0.25 | 0.35 |

The RCₘₐₓ denotes a recovery coefficient calculated based on an SUVₘₐₓ, the RCₘₑₐₙ denotes a recovery coefficient calculated based on an SUVₘₑₐₙ, the Expected RC, the Min RC, and the Max RC denote the expected value, the lower bound, and the upper bound of the RCₘₐₓ or the RCₘₑₐₙ, respectively, and the Sphere Volume denotes a volume of the sphere. More descriptions regarding the SUVₘₐₓ and the SUVₘₑₐₙ may be found in FIG. 9 and related descriptions thereof.

In some embodiments, the processing device 140 may obtain the reference standard from the storage device 150. The reference standard in the storage device 150 may be input by a user in advance or automatically imported by the system.

In some embodiments, the processing device may determine whether the first recovery coefficient satisfies the reference standard based on the RC curves of the phantom image in a plurality of ways. For example, the processing device may determine whether the first recovery coefficient satisfies the reference standard by determining whether the RC curves corresponding to the first recovery coefficient fall within a range of the upper bound, the lower bound, and the lower bound of the RC curves corresponding to the reference standard. Exemplarily, whether the first recovery coefficient satisfies the reference standard may be determined by data on a visualization interface described in FIG. 5. When curves (line graphs) corresponding to the first recovery coefficient are both located between a line graph of the upper bound and a line graph of the lower bound of the RC curves corresponding to the reference standard, the first recovery coefficient satisfies the reference standard.

In some embodiments, the processing device may determine whether a difference between the first recovery coefficient and an expected value in the reference standard satisfies an error preset condition. If the error preset condition is satisfied, it may be determined that the first recovery coefficient satisfies the reference standard, i.e., the RC curves corresponding to the first recovery coefficient fall within the range of the upper bound and lower bound of the RC curves corresponding to the reference standard. The error preset condition may include that an average value of the differences, each of which is between the first recovery coefficient of one of the six spheres and one of the expected values corresponding to the six spheres in the reference standard is less than or equal to 10%. For example, the differences between the first recovery coefficients of the 6 spheres and the expected values corresponding to the 6 spheres in the reference standard are 10%, 7%, 9%, 12%, 8%, and 8% of their expected values, respectively, an average value of 9% is obtained, which satisfies a condition of less than or equal to 10% (i.e., the error preset condition), then the first recovery coefficient is determined to satisfy the reference standard. As another example, each of the differences between the first recovery coefficients of the 6 spheres and the corresponding expected values of the 6 spheres in the reference standard is less than or equal to 10% of a corresponding expected value, which indicates that the average value is also less than or equal to 10% and satisfies the error preset condition.

FIG. 7 is a schematic diagram illustrating an upper bound, a lower bound, an expected value of RCₘₐₓ in a reference standard, and a first recovery coefficient corresponding to RCₘₐₓ satisfying the reference standard according to some embodiments of the present disclosure.

As shown in FIG. 7, curve 711 represents a curve corresponding to RCₘₐₓ of six spheres of a phantom image before being processed by a first processing. Curve 712 represents a curve corresponding to the upper bound of RCₘₐₓ of 6 spheres in a phantom in the reference standard. Curve 713 represents a curve corresponding to RCₘₐₓ of first recovery coefficients of the 6 spheres in the phantom. Curve 714 represents a curve corresponding to expected values of the RCₘₐₓ of the 6 spheres in the phantom in the reference standard. Curve 715 represents a curve corresponding to the lower bound of the RCₘₐₓ of the 6 spheres in the phantom in the reference standard. As shown in FIG. 7, a first recovery coefficient obtained after a plurality of iterations of an RC curve (curve 711) of the phantom image falls between the upper bound of the RCₘₐₓ of the reference standard (curve 712) and the lower bound (curve 715), i.e., the first recovery coefficient satisfies the reference standard. Similarly, the processing device may determine, based on the same manner, whether first recovery coefficient corresponding to each of the RCₘₑₐₙ and the RCₚₑₐₖ satisfy the reference standard.

In some embodiments, in response to determining that the first recovery coefficient does not satisfy the reference standard, the processing device may determine a customized standard as a target normalization standard. The first coefficient not satisfying the reference standard may include any of the RCₘₐₓ, the RCₘₑₐₙ, and the RCₚₑₐₖ not satisfying a range of the upper bound and a range of the lower bound of the RC curve corresponding to the reference standard.

The customized standard refers to a user-defined normalization standard for a standard uptake value.

In some embodiments, the customized standard may be determined based on a user input parameter.

The user input parameter may include a parameter input by the user for determining the customized standard. The user input parameter may be determined based on first recovery coefficients corresponding to the 6 spheres in the phantom.

In some embodiments, the first recovery coefficients corresponding to the 6 spheres in the phantom input by the user may include an RCₘₐₓ, an RCₘₑₐₙ, and an RC_{peak.} In some embodiments, the processing device may determine the user input parameter based on the first recovery coefficient that does not satisfy the reference standard. Exemplarily, referring to FIG. 7, if a curve corresponding to the first recovery coefficient (curve 713) corresponds to an RCₘₐₓ that does not satisfy the reference standard, the user may determine, based on specific values of the 6 spheres on the curve 713, RC values of the 6 spheres as user input parameters. In some embodiments, an RC value may be a value within a neighboring range (e.g., 0.1%, 0.2%, etc.) of the RC values of the spheres on curve 713.

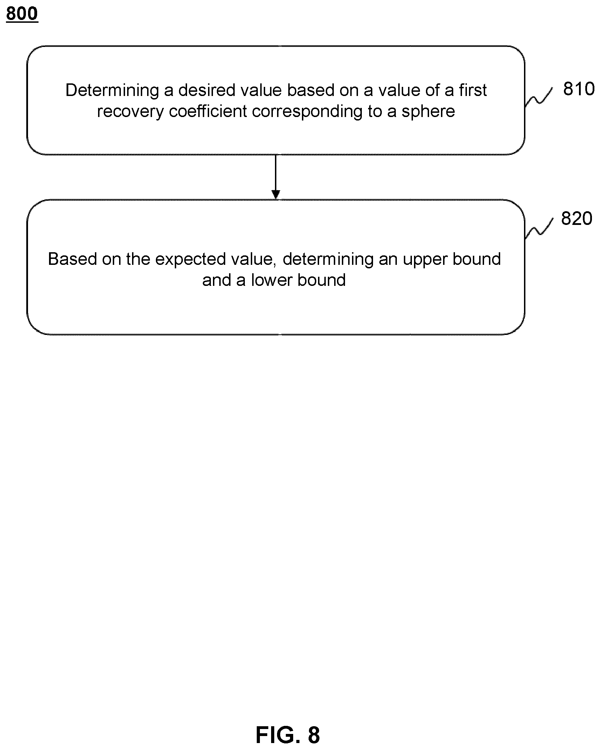

In some embodiments, the customized standard may include a maximum activity value, a mean activity value, and a mean value of a peak activity region including an expected value, an upper bound, and a lower bound, respectively. The processing device determining the customized standard may include determining an expected value based on the first recovery coefficient corresponding to a sphere and determining the upper bound and the lower bound based on the expected value. More relevant descriptions may be found in FIG. 8 and related descriptions thereof.

In some embodiments, the phantom image and an image to be processed are obtained based on the same acquisition condition and the same reconstruction condition. It may be understood that the phantom image and the image to be processed are obtained based on the same acquisition condition and the same reconstruction condition, and then a target processing parameter is determined based on the phantom image and the reference standard or customized standard, which may be used to process the image to be processed.

Exemplarily, the same acquisition condition may include the same acquisition parameters for obtaining the phantom image and the image to be processed, such as an image acquisition duration, a concentration of a radioactive substance infused before the acquisition, a scanning speed, an acquisition mode, a decay correction, etc. The same reconstruction condition may include the same image reconstruction conditions for the phantom image and the image to be processed, such as an image reconstruction algorithm, a matrix size, layer thickness, a count of iterations, etc. More descriptions regarding the acquisition parameter and the reconstruction parameter may be found in FIG. 4.

In 620, a plurality of iterations are performed to obtain a second recovery coefficient, in each iteration, a first processing is performed on the phantom image, and in response to determining that the second recovery coefficient satisfies the customized standard, the target processing parameter is obtained.

The second recovery coefficient refers to an RC parameter obtained when the first processing is iteratively performed on the RC curves of the phantom image to make the normalized RC curves of the phantom image satisfy the normalization standard (the customized standard). The second recovery coefficient differs from the first recovery coefficient in that the count of iterations during the first processing and/or corresponding filter parameters may be different. In some embodiments, based on the data of the EARL V1.0 standard, i.e., the data in Table 1 above as a reference, the customized standard is determined, and the customized standard includes a standard range corresponding to the RCₘₐₓ and the RCₘₑₐₙ. Then the second recovery coefficient may include a maximum recovery coefficient (RCₘₐₓ) and a mean recovery coefficient (RCₘₑₐₙ). In some embodiments, the second recovery coefficient may also be represented by RC curves. Correspondingly, the RC curves corresponding to the second recovery coefficient may also include an RCₘₐₓ curve, and an RCₘₑₐₙ curve. More detailed descriptions regarding determining the customized standard may be found in FIG. 8 and related descriptions thereof.

In some embodiments, filter parameters of a Gaussian filtering process for the RCₘₐₓ curve, the RCₘₑₐₙ curve, and the RCₚₑₐₖ curve corresponding to the second recovery coefficient may be denoted by Filterₘₐₓ, Filterₘₑₐₙ, and Filterₚₑₐₖ, respectively. In some embodiments, other possible filter-related parameters may be included and are not limited herein.

Here, the target processing parameter is a filter parameter when the second recovery coefficient satisfies the customized standard.

In some embodiments, the processing device may obtain the second recovery coefficient by performing the Gaussian filtering process on the RC curves of the phantom image over a plurality of iterations and record one or more filter parameters at each of the plurality of iterations.

In some embodiments, the iteration process is terminated when the second recovery coefficient satisfies the normalization standard, and a filter parameter generated at the current iteration after which the iteration process is terminated is recorded as the target processing parameter.

In some embodiments, the way of determining whether the second recovery coefficient satisfies the normalization standard is similar to the way of determining whether the first recovery coefficient satisfies the reference standard, and more detailed descriptions may be found in related descriptions in operation 610.

In 630, a second processing is performed on the image to be processed based on the target processing parameter to determine a normalized standard uptake value.

In some embodiments, when the second recovery coefficient satisfies the normalization standard, the processing device may perform the second processing on the image to be processed based on the target processing parameter to obtain the normalized standard uptake value.

The second processing refers to the Gaussian filtering process on the image to be processed based on the target processing parameter. In some embodiments, the second processing may include performing the Gaussian filtering process on the image to be processed once based on the target processing parameter.

The normalized standard uptake value refers to a standard uptake value that may be comparable across devices, patients, and time, after normalization, i.e., a standard uptake value that may be quantitatively analyzed after the influence of different devices, acquisition parameters, reconstruction manners, and reconstruction parameters have been eliminated to some extent.

In some embodiments, the processing device may perform the second processing on the image to be processed based on the target processing parameter to determine a normalized image and calculate the normalized standard uptake value based on the normalized image. More relevant descriptions may be found in FIG. 9.

In one or more embodiments of the present disclosure, through a normalization manner of a standard uptake value, the customized standard may be provided when the reference standard fails to meet application needs, to overcome the problem of noncomparability of the standard uptake value due to the difference in models of the devices, reconstruction algorithms, etc. Thus, the standard uptake value is comparable across devices, patients, and time, so that the obtained standard uptake value may be used for clinical analysis to meet clinical needs.

FIG. 8 is a flowchart illustrating an exemplary process for determining a customized standard according to some embodiments of the present disclosure. In some embodiments, process 800 may be performed by the system 200 or the processing device 140. As shown in FIG. 8, process 800 includes the following operations.

In 810, an expected value is determined based on a value of a first recovery coefficient corresponding to a sphere.

In some embodiments, the processing device may determine the expected value based on the value of the first recovery coefficient corresponding to the sphere. The value of the first recovery coefficient may include values under the three definitions of RC_{max,} RCₘₑₐₙ, and RC_{peak.} Correspondingly, the expected value corresponds to values under the three definitions of RCₘₐₓ, RCₘₑₐₙ, and RC_{peak.} The value of the first recovery coefficient may be obtained based on a user input, and more descriptions regarding the value of the first recovery coefficient and the obtaining of the value of the first recovery coefficient based on the user input may be found in FIG. 6.

In some embodiments, the processing device may determine the value of the first recovery coefficient corresponding to the sphere input by the user as the expected value in the customized standard. As an example of determining an expected value of an RCₘₐₓ in the customized standard, RCₘₐₓ of the 6 spheres input by the user based on the value of the first recovery coefficient under the definition of RCₘₐₓ are RCₘₐₓ₁-RCₘₐₓ₆, and the processing device may determine each of the RCₘₐₓ₁-RCₘₐₓ₆ as an expected value of the RCₘₐₓ corresponding to each of the 6 spheres in the customized standard. The manner for determining the expected values of the RCₘₑₐₙ and the RCₚₑₐₖ is similar and is not repeated here.

In 820, based on the expected value, an upper bound and a lower bound are determined.

In some embodiments, the processing device may determine the upper bound and the lower bound of the RC value corresponding to the customized standard based on the expected value in the customized standard. In some embodiments, the processing device may increase or decrease a preset value of the expected value corresponding to the customized standard to determine the upper bound and the lower bound in the customized standard. The preset value may be set in advance based on experience or set by default by the system. Merely by way of example, the preset value may be 5% of the expected value, and when the expected value of the RCₘₐₓ of a sphere in the customized standard is RCₘₐₓ₁, the upper bound of the RCₘₐₓ corresponding to the sphere is RCₘₐₓ₁*(1+5%) and the lower bound is RCₘₐₓ₁*(1-5%). Merely by way of example, Table 2 shows the RCₘₐₓ of the customized standard corresponding to the six spheres.

**Table 2**

| Sphere Volume(ml) | Expected RCₘₐₓ | Min RCₘₐₓ | Max RCₘₐₓ |
|---|---|---|---|
| 26.52 | RCₘₐₓ₁ | RCₘₐₓ₁* (1-5%) | RCₘₐₓ₁* (1+5%) |
| 11.49 | RCₘₐₓ₂ | RCₘₐₓ₂* (1-5%) | RCₘₐₓ₂* (1 +5%) |
| 5.57 | RCₘₐₓ₃ | RCₘₐₓ₃* (1-5%) | RCₘₐₓ₃* (1 +5%) |
| 2.57 | RCₘₐₓ₄ | RCₘₐₓ₄* (1-5%) | RCₘₐₓ₄* (1 +5%) |
| 1.15 | RCₘₐₓ₅ | RCₘₐₓ₅* (1-5%) | RCₘₐₓ₅* (1 +5%) |
| 0.52 | RCₘₐₓ₆ | RCₘₐₓ₆* (1-5%) | RCₘₐₓ₆* (1 +5%) |

Expected RCₘₐₓ, Min RCₘₐₓ, and Max RCₘₐₓ denote the expected value, the lower bound and the upper bound of the RCₘₐₓ of the customized standard, respectively, and Sphere Volume denotes the volume of the sphere. The expected value, the upper bound, and the lower bound under the definition of RCₘₑₐₙ in the customized standard and the determination manner of which are similar to determining the expected value, the upper bound, and the lower bound under the definition of RCₘₐₓ, which is not repeated here.

In some embodiments, the processing device may also determine the customized standard based on a standard determination model including determining the customized standard by processing a first recovery coefficient, a reference standard, a device parameter, an acquisition condition, and a reconstruction condition through the standard determination model. The standard determination model is a machine learning model, such as a neural network model, a deep neural network, other customized models, etc.

In some embodiments, an input of the standard determination model may include the first recovery coefficient, the reference standard, the device parameter, the acquisition parameter, the reconstruction parameter, etc. The device parameter, the acquisition parameter, and the reconstruction parameter may be determined based on a device (e.g., medical imaging device 110) that obtains a phantom image and an image to be processed. More descriptions regarding the first recovery coefficient, the reference standard, the acquisition parameter, and the reconstruction parameter may be found elsewhere in the present disclosure, such as in FIG. 3, FIG. 6, etc.

In some embodiments, an output of the standard determination model may include the customized standard. The customized standard output by the standard determination model may be represented in the form of a matrix. Each column of the matrix represents a different definition (e.g., RCₘₐₓ, RCₘₑₐₙ), and each row represents the expected value, the upper bound, and the lower bound under each definition.

In some embodiments, the standard determination model may be obtained by training an initial standard determination model without setting parameters based on training samples with labels. The training samples may be obtained based on historical data including a plurality of reference standards, a plurality of sets of historical first recovery coefficients obtained by a plurality of iterations of historical Gaussian filtering on RC curves of the phantom image, a device parameter when performing historical image acquisition and historical reconstruction, a historical acquisition condition, a historical reconstruction condition, etc. The labels of the training samples may be an upper bound, a lower bound, and an expected value corresponding to each of the plurality of sets of historical first recovery coefficients, which may be obtained based on the experience by manual labeling. For example, values corresponding to 6 spheres in each of the plurality of sets of historical first recovery coefficients may be determined as expected values of the 6 spheres, and each of the expected values may be added or subtracted from preset values as the upper bound and the lower bound, respectively, for determining the labels.

An exemplary training process may include inputting a plurality of labeled training samples into an initial standard determination model, updating the parameters of the initial standard determination model through training until a condition is satisfied, e.g., a loss function is less than a threshold, convergence is achieved, or a training period reaches a threshold, etc., and obtaining the trained standard determination model.

In one or more embodiments of the present disclosure, the customized standard determined by the machine learning model is more accurate, and normalization of the standard uptake value may be better, thereby improving efficiency and saving costs.

In some embodiments, the processing device may send the reference standard and the customized standard to the terminal device 130 to determine a target normalization standard by user selection.

In some embodiments, the target normalization standard may also be determined based on the processing device. For example, the processing device may perform a first processing on the RC curves of the phantom image for a plurality of iterations based on Gaussian filtering, compare the RC curves of the phantom image after iterations with the reference standard and the customized standard, determine whether an error between the RC curves of the phantom image and each of the expected values of the reference standard and an expected value of the customized standard satisfies an error preset condition, and select a standard among the reference standard and the customized standard that satisfies the error preset condition as the target normalization standard. More detailed descriptions regarding the error preset condition may be found in related descriptions in FIG. 6. In some embodiments, the processing device may also separately determine whether RC curves of the iterated phantom image fall within a range of the upper bound and the lower bound of the reference standard or the customized standard, select the standard, among the reference standard and the customized standard, in which the RC curves of the phantom image falls within the range of the upper bound and the lower bound as the target normalization standard.

In one or more embodiments of the present disclosure, a customized standard determined based on the method described above may solve the problem that the reference standard cannot meet the clinical need in some cases so that the standard uptake value may be normalized successfully.

FIG. 9 is a flowchart illustrating an exemplary process for determining a normalized standard uptake value according to some embodiment of the present disclosure. In some embodiments, process 900 may be performed by a processing device. As shown in FIG. 9, process 900 includes the following operations.

In 910, a normalized image is determined by performing a second processing on an image to be processed based on a target processing parameter.

The normalized image is the image to be processed after the second processing. In some embodiments, the processing device may determine the normalized image by performing the second processing on the image to be processed based on the target processing parameter obtained when RC curves of the phantom image satisfy a normalization standard. More descriptions regarding the second processing, the image to be processed, and the target processing parameter may be found in the relevant descriptions hereinabove, such as FIG. 3, FIG. 6, etc.

In 920, a normalized standard uptake value is determined based on the normalized image.

A standard uptake value (SUV) includes SUVₘₐₓ, SUVₘₑₐₙ, and SUVₚₑₐₖ. In some embodiments, the standard uptake value of the image to be processed may be obtained based on an image-based visual judgment of the image to be processed. For example, the standard uptake value of the image to be processed may be obtained based on machine vision-based image recognition and processing. SUVₘₐₓ is the maximum value of SUVs of all pixels in a region of interest (ROI), SUVₘₑₐₙ is an average value of SUVs for all pixels in the ROI, SUVₚₑₐₖ is the maximum value of the average value of SUVs for pixels in a small region (e.g., within 1cm³) inside the ROI.

In some embodiments, the processing device may further determine, based on the standard uptake value, a metabolic tumor volume (MTV) and a total lesion glycolysis (TLG) in a lesion area. Exemplarily, the MTV may be determined by a percentage threshold manner, e.g., MTV=40%SUVₘₐₓ, and the TLG may be determined based on the MTV of a lesion area and the SUVₘₑₐₙ within the lesion area, e.g., TLG=MTV×SUVₘₑₐₙ.

In some embodiments, to achieve the standard uptake value comparable across devices, patients, and time to satisfy the clinical needs, the processing device may determine, based on an image to be processed after normalization, a normalized standard uptake value, which may be denoted respectively as u.SUVₘₑₐₙ, u.SUVₘₐₓ, and u.SUVₚₑₐₖ. Further, the processing device may update, based on the u.SUVₘₑₐₙ, the u.SUVₘₐₓ, and the u.SUVₚₑₐₖ, clinical neonatal indicators MTV and TLG synchronously to obtain normalized MTV and TLG, which may be expressed as u.MTV and u.TLG, respectively. The u.MTV and the u.TLG are determined in the same way as MTV and TLG, which may be seen above.

The processing device may transmit the MTV, TLG, and/or u.MTV and u.TLG to the terminal device 130 for clinical application by the user.

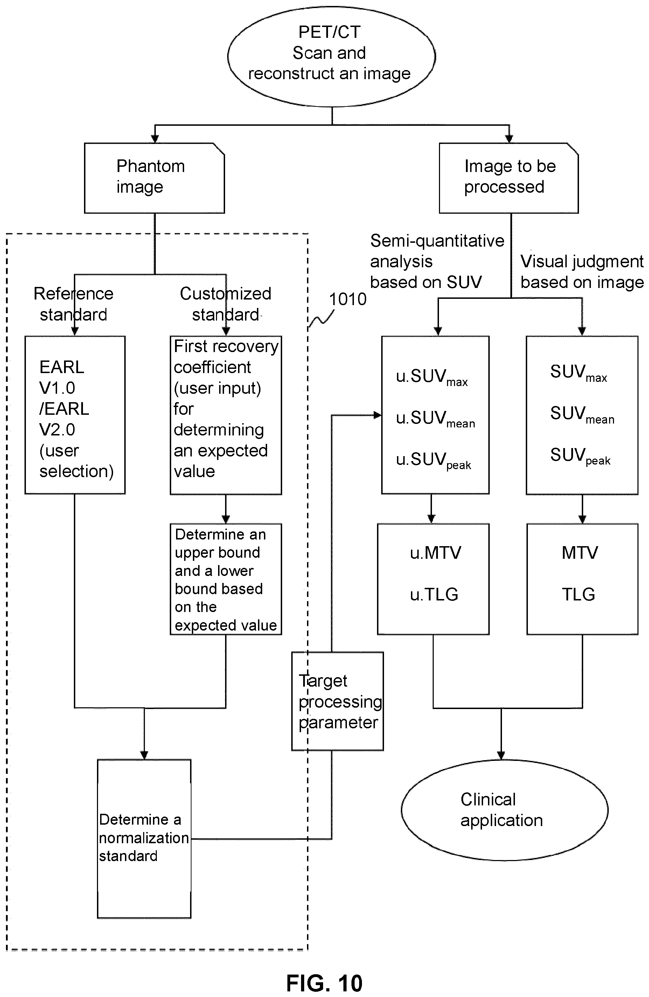

FIG. 10 is a schematic diagram illustrating a semi-quantitative analysis based on a normalized standard uptake value according to some embodiments of the present disclosure.

As shown in FIG. 10, a processing device may scan and reconstruct an image based on a PET/CT device, including scanning and reconstructing a phantom and an object to be processed (i.e., an object to be subjected to clinical diagnosis) to obtain a phantom image and an image to be processed. More descriptions regarding obtaining the phantom image and the image to be processed may be found elsewhere in the present disclosure, such as FIG. 1, FIG. 2, FIG. 3, etc.

In some embodiments, when a semi-quantitative analysis of a standard uptake value (SUV) is performed, the processing device may determine a normalization standard and determine a normalized SUV based on the normalization standard. Detailed descriptions are as follows.

Continuing to refer to FIG. 10, the processing device may determine a target processing parameter based on operations shown in 1010.

As shown in FIG. 10, operation 1010 includes iteratively performing a first processing on the phantom image to obtain a first recovery coefficient. The processing device may determine whether the first recovery coefficient satisfies a reference standard (i.e., an EARL standard including at least one of an EARL V1.0 standard and an EARL V2.0 standard) or a customized standard, determine a standard among the reference standard or a customized standard satisfied by the first recovery coefficient as the normalization standard. More descriptions regarding the first recovery coefficient, the reference standard, the customized standard, and the determination of the normalization standard may be found elsewhere in the present disclosure, such as in FIG. 3, FIG. 6, etc.

After determining the normalization standard, the processing device may determine the target processing parameter based on the normalization standard, including iteratively performing a first processing on the phantom image to obtain the second recovery coefficient and a processing parameter, and when the second recovery coefficient satisfies the normalization standard, the filter parameter (the processing parameter) of the first processing performed in the last iteration is determined as the target processing parameter of the second processing on the image to be processed. The processing device may process the image to be processed based on the target processing parameter, obtain the normalized image, determine the normalized SUV based on the normalized image, synchronously modify MTV and TLG, and correspondingly generate u.MTV and u.TLG, and apply the generated u.MTV and u.TLG to clinical applications. More descriptions regarding the second recovery coefficient, the processing parameter, the target processing parameter, etc. may be found elsewhere in the present disclosure, such as FIG. 6, etc.

In some embodiments, when the semi-quantitative analysis of the SUV is not required, the processing device may determine, based on the image to be processed, the SUV of the image to be processed (including SUVₘₐₓ, SUVₘₑₐₙ, and SUVₚₑₐₖ) of the image to be processed through an image-based visual judgment, then determine the MTV and the TLG based on the SUV, and clinically apply the obtained MTV and TLG.

When performing normalization on the SUV using the EARL standard, it is not possible to realize normalization on the SUVₘₑₐₙ, the SUVₘₐₓ, and the SUVₚₑₐₖ at the same time, due to varying degrees of missing for an RC range in both EARL V1.0 standard and EARL V2.0 standard, making a low value in an application of SUV in efficacy assessment and intergroup comparison.

In view of the foregoing, some embodiments of the present disclosure provide supplemental definitions of the reference standard for obtaining a normalization factor that may be used to perform normalization on the image to be processed simultaneously under the three definitions (RCₘₐₓ, RCₘₑₐₘ, and RCₚₑₐₖ). In some embodiments, an image processing result may include the normalized standard uptake value. In some embodiments, the processing device may determine the normalization factor based on the phantom image and the reference standard, and the normalization factor is used to perform a second processing (e.g., normalization process) on the image to be processed to determine the normalized standard uptake value. The manner described in the embodiment may realize that, in the case where the RC range in the EARL standard is partially missing, images to be processed obtained based on different scanning devices, different patients, and different times may be subjected to the normalization under SUVₘₑₐₙ, SUVₘₐₓ, SUVₘₐₓ, and SUVₚₑₐₖ, which makes the SUV comparable across devices, patients, and time, and thus enables benign and malignant tumor identification and efficacy evaluation.

FIG. 11 is a flowchart illustrating an exemplary process for determining a normalized standard uptake value according to some embodiments of the present disclosure. In some embodiments, the process 1100 may be performed by the processing device 140 or the system 200. As shown in FIG. 11, process 1100 includes the following operations.

In 1110, a target processing parameter is determined based on a phantom image and a reference standard, wherein the target processing parameter is a normalization factor.

The reference standard may include an EARL V1.0 standard and an EARL V2.0 standard. The reference standard may include one or more determined standard parameters. The standard parameter refers to one or more types of parameters used to evaluate a characteristic of an RC value. The standard parameter may include but is not limited to, one of a maximum recovery coefficient (also referred to as RCₘₐₓ), a mean recovery coefficient (also referred to as RCₘₑₐₙ), and a regional mean value of a peak recovery coefficient (also referred to as the RCₚₑₐₖ), which may be used as a standard to be satisfied by RC curves corresponding to a normalized phantom image. More descriptions regarding the phantom image, the reference standard, and the target processing parameter may be found in the relevant descriptions hereinabove, such as FIG. 3, FIG. 6, etc.

The RCₘₐₓ refers to the reference standard used for the normalization of SUVₘₐₓ, the RCₘₑₐₙ refers to the reference standard used for the normalization of SUVₘₑₐₙ, and the RCₚₑₐₖ refers to the reference standard used for the normalization of SUVₚₑₐₖ. The SUVₘₐₓ refers to the maximum value of the SUV of all pixels in a region of interest (ROI), the SUVₘₑₐₙ refers to a mean value of the SUV of all pixels in the ROI, and SUVₚₑₐₖ refers to the maximum value of mean values of the SUV of pixels in a small internal region (e.g., within 1 cm³) of the ROI.

Standard parameters in the EARL V1.0 standard include the RCₘₐₓ and the RCₘₑₐₙ. As shown in Table 3 below, the EARL V1.0 standard may include 6 spheres with different diameters, and upper bounds (i.e., Max RC), lower bounds (i.e., Min RC), and expected values (i.e., Expected RC) of RCₘₐₓ and RCₘₑₐₙ corresponding to each of the 6 spheres.

**Table 3**

| Sphere Diameter (mm) | RCₘₐₓ | | | RCₘₑₐₙ | | |
|---|---|---|---|---|---|---|
| | Expected RC | Min RC | Max RC | Expected RC | Min RC | Max RC |
| 37 | 0.98 | 0.88 | 1.08 | 0.77 | 0.71 | 0.83 |
| 28 | 0.95 | 0.85 | 1.05 | 0.73 | 0.67 | 0.79 |
| 22 | 0.89 | 0.77 | 1.01 | 0.66 | 0.59 | 0.73 |
| 17 | 0.84 | 0.75 | 0.94 | 0.6 | 0.53 | 0.68 |
| 13 | 0.63 | 0.51 | 0.74 | 0.45 | 0.38 | 0.52 |
| 10 | 0.38 | 0.29 | 0.46 | 0.3 | 0.25 | 0.35 |

Standard parameters in the EARL V2.0 standard include RCₘₐₓ, RCₘₑₐₙ, and RC_{peak.} As shown in Table 4 below, the EARL V2.0 standard may include diameters of 6 spheres, and standard ranges of RCₘₑₐₙ, RCₘₐₓ, and RCₚₑₐₖ corresponding to each of the 6 spheres, i.e., ranges from lower bound to upper bound.

**Table 4**

| Sphere Diameter(mm) | Proposed PC bandwidth | | |
|---|---|---|---|
| | RCₘₑₐₙ | RCₘₐₓ | RCₚₑₐₖ |
| 37 | 0.85-1.00 | 1.05-1.29 | 0.99-1.07 |
| 28 | 0.82-0.97 | 1.01-1.26 | 0.95-1.07 |
| 22 | 0.80-0.99 | 1.01-1.32 | 0.90-1.09 |
| 17 | 0.76-0.97 | 1.00-1.38 | 0.75-0.99 |
| 13 | 0.63-0.86 | 0.85-1.22 | 0.45-0.69 |
| 10 | 0.39-0.61 | 0.52-0.88 | 0.27-0.41 |

In some embodiments, the processing device may obtain the reference standard by accessing a phantom database of NEMA IQ. More detailed descriptions regarding the phantom of NEMA IQ may be found in related descriptions above.

The normalization factor refers to a parameter used to subject an image to be processed to a second processing (i.e., normalization), i.e., a filter parameter.

The normalization factor may include at least one of a first factor, a second factor, and a third factor. The first factor is a parameter used to subject the image to be processed to the second processing to determine a parameter of a normalized SUVₘₑₐₙ. That is, a parameter used to subject the image to be processed to Gaussian filtering to determine the filter parameter of the normalized SUVₘₑₐₙ, which may be denoted as Filterₘₑₐₙ. Similarly, the second factor is a filter parameter used to subject the image to be processed to the second processing to determine a normalized SUVₘₐₓ, which may be denoted as Filterₘₐₓ. The third factor refers to a filter parameter used to subject the image to be processed to the second processing to determine a normalized SUVₚₑₐₖ SUVₚₑₐₖ, which may be denoted as Filterₚₑₐₖ.

In some embodiments, the processing device may process the phantom image by Gaussian filtering with a preset parameter (a preset size of a filter), obtain a processed phantom image, and then calculate an RC curve corresponding to the processed phantom image. The preset parameter (the preset size of the filter) may be a default value, an empirical value, and the like.

In some embodiments, the processing device may determine, based on the phantom image, an actual radioactivity ratio of the spheres and a background region of the phantom and determine, based on a concentration ratio of the spheres and the developer infused into the background region of the phantom, an ideal radioactivity ratio of the spheres and the background region of the phantom. Then the processing device may calculate, based on the actual radioactivity ratio and the ideal radioactivity ratio, a recovery coefficient for each of the spheres and obtain an RC curve corresponding to the phantom image based on the recovery coefficient of each of the spheres. More descriptions regarding the recovery coefficient may be found in FIG. 3 and related descriptions thereof. An abscissa of each of the spheres in the RC curve is a diameter of each of the spheres, and an ordinate is a recovery coefficient corresponding to each of the spheres. In some embodiments, the abscissa of each of the spheres in the RC curve may also be expressed as a volume of each of the spheres, and the volume may be calculable based on the diameter.

The processing device may determine whether RC curves corresponding to a phantom image after the first processing satisfy corresponding recovery preset conditions and determine a filter parameter that satisfies the recovery preset conditions to be a normalization factor, i.e., a target processing parameter.

The recovery preset conditions refer to conditions to be satisfied by the RC curves corresponding to the phantom image after the first processing. For example, the recovery preset conditions may include that an absolute percentage difference between an RC value of each of the spheres in the RC curves corresponding to the phantom image after the first processing and an expected value of the RC value of a corresponding sphere in the reference standard satisfies an error preset condition. More descriptions regarding the expected value and the error preset condition may be found in FIG. 6 and related descriptions thereof. As another example, the recovery preset conditions may be that the RC value of each of the spheres in the RC curves corresponding to the phantom image after the first processing falls within a range from an upper bound to a lower bound of the RC value of the corresponding sphere in the reference standard.

The recovery preset conditions may include conditions that need to be satisfied by RC curves under three definitions (RCₘₐₓ, RCₘₑₐₙ, and RCₚₑₐₖ), which may be denoted as RCₘₐₓ recovery preset conditions, RCₘₑₐₙ recovery preset conditions, and RCₚₑₐₖ recovery preset conditions, respectively.

Merely by way of example, the processing device may process the phantom image through a Gaussian filter with a preset parameter and obtain a processed phantom image. Then the processing device may calculate an RCₘₑₐₙ curve corresponding to the processed phantom image and determine whether the corresponding RCₘₑₐₙ curve of the processed phantom image satisfies a corresponding RCₘₑₐₙ recovery preset condition. When the RCₘₑₐₙ curve satisfies the RCₘₑₐₙ recovery preset conditions, the processing device may record the filter parameter at this time as Filterₘₑₐₙ, i.e., the first factor.

The manner in which the processing device determines the second factor and the third factor is similar to the manner in which the processing device determines the first factor, and the manner in which the processing device determines the second factor and the third factor may refer to descriptions regarding the manner in which the processing device determines the first factor hereinabove.

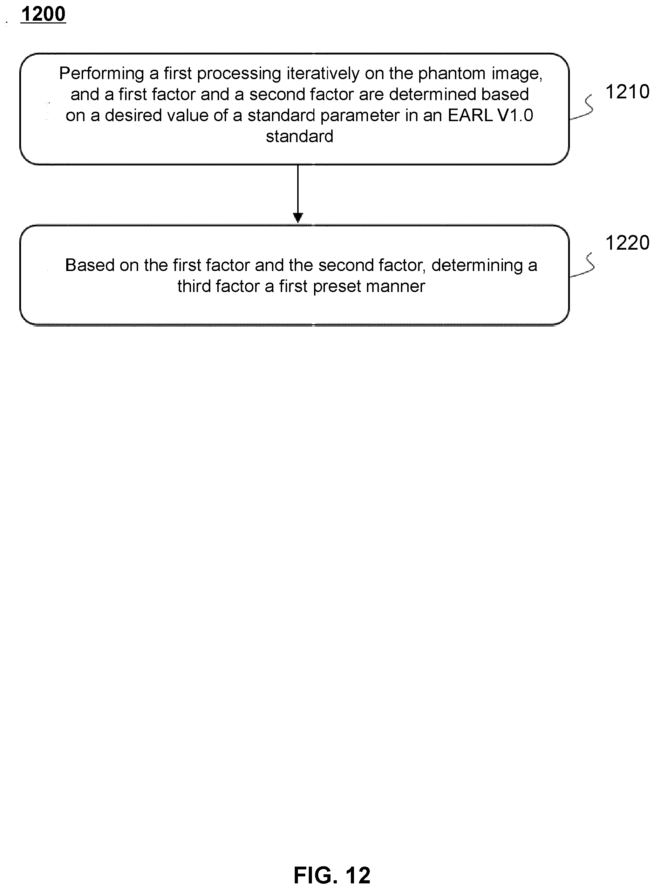

In some embodiments, the processing device may perform the first processing iteratively on the phantom image and determine the first factor and the second factor based on an expected value of a standard parameter in the EARL V1.0 standard and based on the first factor and the second factor, determine a third factor by a first preset manner. More descriptions regarding the above embodiments may be found in FIG. 12 and related descriptions thereof.

In some embodiments, the processing device may also calculate the expected value of the standard parameter based on an upper bound and a lower bound of the standard parameter in the EARL V2.0 standard by a second preset manner, perform the first processing iteratively on the phantom image, and determine at least one of the first factor, the second factor, and the third factor based on the expected value of the standard parameter. More descriptions regarding the above embodiments may be found in FIG. 15 and related descriptions thereof.

In 1120, a second processing is performed on the image to be processed using a normalization factor to determine a normalized standard uptake value.

In some embodiments, the processing device may perform a Gaussian filtering process on the image to be processed based on the normalization factor to obtain the normalized standard uptake value. More descriptions regarding the image to be processed and the normalized standard uptake value may be found elsewhere in the present disclosure, such as FIG. 3, FIG. 9, etc.

It is worth stating that, the accuracy and repeatability of a standard uptake value before normalization are reduced due to the effects of an examination process, an image acquisition and reconstruction manner, etc. Some embodiments of the present disclosure determine the normalization factor based on the phantom image and the reference standard and perform the second processing (normalization processing), based on the normalization factor, on the image to be processed to obtain the normalized standard uptake value. By using the same acquisition condition and the same reconstruction condition for obtaining the phantom image and the image to be processed, and by controlling the variables to reduce the amount of computation, ultimately, an SUV that is comparable across devices, patients, and time is obtained, thereby improving the application value of the SUV in the efficacy assessment, intergroup comparison, etc.

In some embodiments, the processing device may generate a normalized MTV and a normalized TLG based on the normalized standard uptake value, which reflects the tumor metabolic activity and tumor metabolic volume. More descriptions regarding MTV and TLG may be found in FIG. 9, FIG. 10, etc.

FIG. 12 is an exemplary flowchart for determining a normalization factor based on an EARL V1.0 standard according to some embodiments of the present disclosure. In some embodiments, process 1200 may be performed by a processing device. As shown in FIG. 12, process 1200 may include the following operations.

In 1210, a first processing is performed iteratively on the phantom image, and a first factor and a second factor are determined based on an expected value of a standard parameter in an EARL V1.0 standard.

More descriptions regarding the standard parameter, the first factor, and the second factor in the EARL V1.0 standard may be found in FIG. 11 and related descriptions thereof.

Understandably, since there lacks an upper bound, a lower bound, and an expected value of RCₚₑₐₖ in the EARL V1.0 standard, the processing device may only perform a second processing (normalization processing) iteratively on a phantom image based on the standard parameter (i.e., upper bounds, lower bounds and expected values of RCₘₑₐₙ and RCₘₐₓ) in the EARL V1.0 standard to determine the first factor and the second factor, but it is difficult to obtain the third factor.

The manner in which the processing device performs a first processing iteratively on the phantom image and determines the first factor and the second factor based on the expected value of the standard parameter in the EARL V1.0 standard is similar to the manner in which the processing device determines a normalization factor based on the phantom image and the reference standard, and the manner in which the processing device performs the first processing iteratively on the phantom image and determines the first factor and the second factor based on the expected value of the standard parameter in the EARL V1.0 standard may refer to the manner in which the processing device determines the normalization factor based on the phantom image and the reference standard in FIG. 11.

In 1220, based on the first factor and the second factor, a third factor is determined by a first preset manner.

The first preset manner refers to a method for determining the third factor. For example, the first preset manner may include determining the third factor based on device parameters, the first factor, and the second factor through operational experience.

In some embodiments, the first preset manner may include determining the third factor based on a maximum value of the first factor and a maximum value of the second factor. For example, the processing device may sort the first factor and the second factor in ascending, with the largest value thereof determined directly as the third factor.

It is worth stating that it was found through a modal test based on the EARL V2.0 standard that when the first processing of the phantom image is performed to satisfy a range of the RCₚₑₐₖ in the EARL V2.0 standard, at that time, filter parameters are a maximum value of Filterₘₐₓ and a maximum value of Filterₘₑₐₙ. Therefore, some embodiments of the present disclosure determine the third factor in the EARL V1.0 standard based on a maximum value of the first factor and a maximum value of the second factor, which is conducive to reducing the amount of calculation.

In some embodiments of the present disclosure, the first processing is performed iteratively on the phantom image, and the first factor and the second factor are determined based on the expected value of the standard parameter in the EARL V1.0 standard. The third factor is determined based on the first factor and the second factor, where the third factor is obtained in the case of the EARL V1.0 standard lacking the upper bound, the lower bound, and the expected value of RCₚₑₐₖ, thus enabling performing the second processing on the image to be processed to obtain normalized standard uptake values of three definitions.

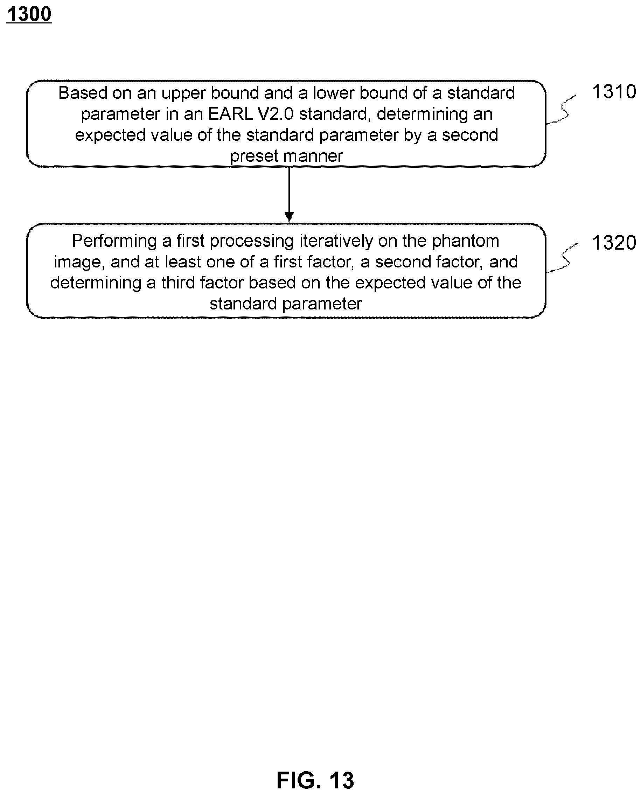

FIG. 13 is a flowchart illustrating an exemplary process for determining a normalization factor based on an EARL V2.0 standard according to some embodiments of the present disclosure. In some embodiments, process 1300 may be performed by a processing device. As shown in FIG. 13, process 1300 may include the following operations.

In 1310, based on an upper bound and a lower bound of a standard parameter in the EARL V2.0 standard, an expected value of the standard parameter is determined by a second preset manner.

More descriptions regarding the standard parameter in the EARL V2.0 standard may be found in FIG. 11 and related descriptions thereof.

It is understood that, due to the lack of expected values of RCₘₑₐₙ, RCₘₐₓ, and RCₚₑₐₖ in the EARL V2.0 standard, the processing device may not perform a second processing iteratively on a phantom image directly based on the standard parameter in the EARL V2.0 standard to determine a first factor, a second factor, and a third factor. Thus, the processing device may first obtain the expected values of RCₘₑₐₙ, RCₘₐₓ, and RCₚₑₐₖ based on the standard parameter in the EARL V2.0 standard, and then further determine the first factor, the second factor, and the third factor.

The expected value of the standard parameter refers to a preferred value of a recovery coefficient (RC).

The second preset manner refers to a method for determining the expected value of the standard parameter. For example, the second preset manner may include determining the expected value based on device parameters, an upper bound, and a lower bound of the standard parameter in the EARL V2.0 standard through operational experience.

In some embodiments, the second preset manner may include determining the expected value based on an average value of the upper bound and the lower bound of the standard parameter. For example, the processing device may determine the average value of the upper bound and the lower bound of RCₘₑₐₙ and designate the average value of the upper bound and the lower bound of RCₘₑₐₙ as the expected value of the RCₘₑₐₙ.

It is worth stating that by calculating standard data in the EARL V1.0 standard shown in Table 3 above, it is found that an expected value of a standard parameter in the EARL V1.0 standard is approximately the average value of the upper bound and the lower bound of the standard parameter.

As shown in above Table 3, the upper bound of the RCₘₑₐₙ of a sphere with a diameter of 37mm is 0.83, the lower bound is 0.71, the average value of the upper bound and the lower bound is (0.83+0.71)/2=0.77, and in above Table 3, an expected value of RCₘₑₐₙ of the sphere with a diameter of 37 mm is also 0.77. Referring again to above Table 3, the upper bound of RCₘₐₓ of the sphere with a diameter of 37 mm is 1.08 and the lower bound is 0.88, and the average value of the upper bound and the lower bound is (1.08+0.88)/2=0.98, and an expected value of the RCₘₐₓ of the sphere with a diameter of 37mm in above Table 3 is also 0.98. Based on this, the processing device may average the upper bound and the lower bound of each standard parameter in the EARL V2.0 standard and take the average of the upper bound and the lower bound of each standard parameter as a corresponding expected value of each standard parameter, which not only enriches the numerical content of the standard parameter in the EARL V2.0 standard but also contributes to the reduction of calculation.

In 1320, a first processing is performed iteratively on the phantom image, and at least one of a first factor, a second factor, and a third factor is determined based on the expected value of the standard parameter.

The manner in which the processing device performs the first processing iteratively on the phantom image and determines at least one of the first factor, the second factor, and the third factor based on the expected value of the standard parameter in the EARL V2.0 standard is similar to the manner in which the processing device determines a normalization factor based on the phantom image and a reference standard. The manner in which the processing device performs the first processing iteratively on the phantom image and determines at least one of the first factor, the second factor, and the third factor based on the expected value of the standard parameter in the EARL V2.0 standard may refer to the manner in which the processing device determines the normalization factor based on the phantom image and the reference standard in FIG. 11.

Some embodiments of the present disclosure may quickly and conveniently supplement missing expected values of RCₘₑₐₙ, RCₘₐₓ, and RCₚₑₐₖ in the EARL V2.0 standard using the upper bound and the lower bound of the standard parameter in the EARL V2.0 standard, thus enriching the numerical content of the standard parameter in the EARL V2.0 standard. Additionally, the expected value of the standard parameter is also used to determine the first factor, the second factor, and the third factor, to realize the normalization of the image to be processed and obtain a normalized standard uptake value.

It should be noted that the foregoing descriptions of the processes (e.g., processes 300, 600, etc.) are intended to be exemplary and illustrative only, and do not limit the application scope of the present disclosure. For those skilled in the art, various corrections and changes may be made to the processes under the guidance of the present disclosure. However, these corrections and changes remain within the scope of the present disclosure.

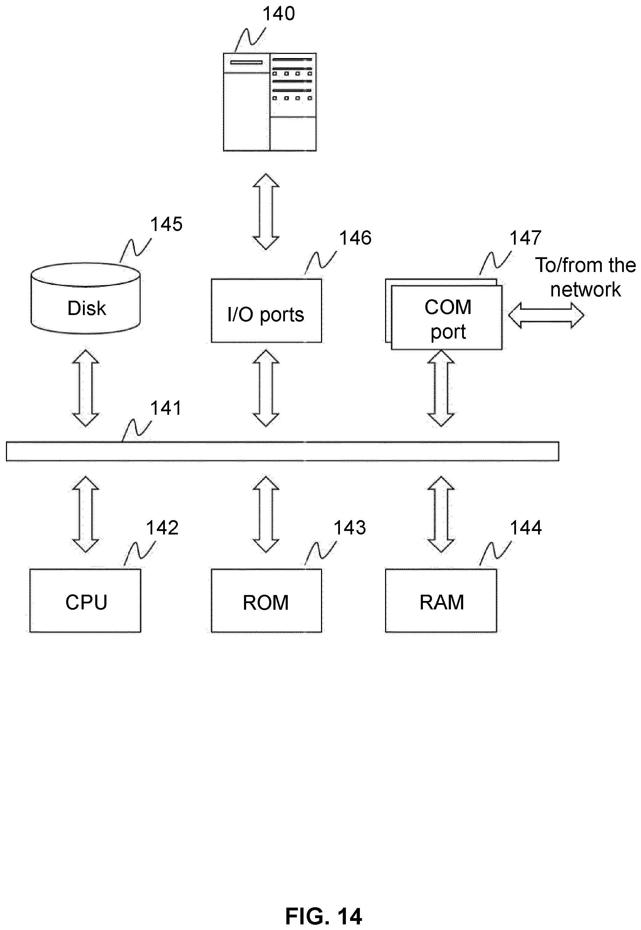

FIG. 14 is a schematic diagram illustrating a structure of a computer that may realize all or part of the functions of a processing device 140 of an image processing system according to some embodiments of the present disclosure. Functions or a part of the functions of the processing device 140 may be realized by a computer via hardware, a software program, firmware, or a combination thereof. While only one computer is shown for convenience, functions of the computer related to the processing device 140 as described herein may be implemented in a distributed manner across a plurality of similar platforms to distribute the processing load. In some embodiments, the computer may be a general-purpose computer or a computer with a specific purpose.

As shown in FIG. 14, the processing device 140 may include a COM port 147, and the COM port may be connected with or from a network to facilitate data communication. The processing device 140 may also include a central processing unit (CPU) 142 including one or more processors and is used to execute program instructions. A computer platform may include an internal communication bus 141 and data memory (e.g., disk 145, read-only memory (ROM) 143, random access memory (RAM) 144). The data memory is used to store various data files processed and/or transmitted by the computer, different forms of programs, program instructions that may be executed by the CPU 142, etc. The processing device 140 may also include input/output (I/O) ports 146, which are used for input or output flows between the processing device 140 and other components in the image processing system (e.g., the terminal device 130).

Having thus described the basic concepts, it may be rather apparent to those skilled in the art after reading this detailed disclosure that the foregoing detailed disclosure is intended to be presented by way of example only and is not limiting. Although not explicitly stated here, those skilled in the art may make various modifications, improvements, and amendments to the present disclosure. These alterations, improvements, and modifications are intended to be suggested by this disclosure.

Moreover, certain terminology has been used to describe embodiments of the present disclosure. For example, the terms "one embodiment," "an embodiment," and/or "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of the present disclosure are not necessarily all referring to the same embodiment. In addition, some features, structures, or characteristics of one or more embodiments in the present disclosure may be properly combined.

Furthermore, the recited order of processing elements or sequences, or the use of numbers, letters, or other designations, therefore, is not intended to limit the claimed processes and methods to any order except as may be specified in the claims. Although the above disclosure discusses some embodiments of the invention currently considered useful by various examples, it should be understood that such details are for illustrative purposes only, and the additional claims are not limited to the disclosed embodiments.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that object of the present disclosure requires more features than the features mentioned in the claims.

In some embodiments, the numbers expressing quantities or properties used to describe and claim certain embodiments of the present disclosure are to be understood as being modified in some instances by the term "about," "approximate," or "substantially." For example, "about," "approximate" or "substantially" may indicate ±20% variation of the value it describes, unless otherwise stated. Correspondingly, in some embodiments, the numerical parameters used in the specification and claims are approximations, which approximations can be varied depending on the desired characteristics of the individual embodiment. In some embodiments, the numerical parameters should take into account the specified number of valid digits and employ general place-keeping. While the numerical domains and parameters used to confirm the breadth of their ranges in some embodiments of the present disclosure are approximations, in specific embodiments such values are set to be as precise as practicable.

## Claims

1. A PET image processing method, comprising:
obtaining (310)
a phantom image and a normalization standard, wherein the normalization standard includes a reference standard;
performing a first processing on the phantom image;
generating a first recovery coefficient of a processed phantom image generated by performing the first processing on the phantom image;
determining whether the first recovery coefficient satisfies the normalization standard;
in response to determining that the first recovery coefficient satisfies the normalization standard, obtaining a target processing parameter based on the phantom image and the reference standard, wherein the target processing parameter is a normalization factor; and
determining an image processing result by processing an image to be processed based on the target processing parameter;
wherein the image processing result includes a normalized standard uptake value; and the determining an image processing result by processing an image to be processed based on the target processing parameter includes:
performing a second processing on the image to be processed using the normalization factor to determine the normalized standard uptake value.

2. The method of claim 1, wherein the normalization standard further includes a customized standard; the image processing result includes a normalized standard uptake value; and the determining an image processing result by processing an image to be processed based on the target processing parameter includes:
in response to determining that the first recovery coefficient does not satisfy the reference standard, determining the customized standard as a target normalization standard; wherein the customized standard is determined by obtaining a user input parameter, and the user input parameter is determined based on a value of the first recovery coefficient corresponding to a sphere in a phantom;
obtaining a second recovery coefficient by performing the first processing iteratively on the phantom image;
obtaining the target processing parameter in response to determining that the second recovery coefficient satisfies the customized standard, wherein the target processing parameter is a processing parameter when the second recovery coefficient satisfies the customized standard;
performing the second processing on the image to be processed based on the target processing parameter; and
determining the normalized standard uptake value.

3. The method of claim 2, the customized standard including a maximum recovery coefficient, a mean recovery coefficient, and a regional mean value of a peak recovery coefficient, wherein each of the maximum recovery coefficient, the mean recovery coefficient, and the regional mean value of the peak recovery coefficient includes an expected value, an upper bound, and a lower bound, respectively; and the customized standard is determined by:
determining the expected value based on the value of the first recovery coefficient corresponding to the sphere; and
determining the upper bound and the lower bound based on the expected value.

4. The method of claim 2, wherein the reference standard includes at least one of an EARL V1.0 standard and an EARL V2.0 standard.

5. The method of claim 2, wherein the phantom image and the image to be processed are obtained based on a same acquisition condition and a same reconstruction condition.

6. The method of claim 1, wherein the reference standard includes an EARL V1.0 standard, the normalization factor includes at least one of a first factor, a second factor, or a third factor; and the determining the target processing parameter based on the phantom image and the reference standard includes:
performing the first processing iteratively on the phantom image;
determining the first factor and the second factor based on an expected value of a standard parameter in the EARL V1.0 standard, wherein the standard parameter in the EARL V1.0 standard includes a maximum recovery factor and an average recovery factor; and
determining the third factor by a first preset manner based on the first factor and the second factor, and the first preset manner includes determining the third factor based on a maximum value of the first factor and the second factor.

7. The method of claim 1, wherein the reference standard includes an EARL V2.0 standard, the normalization factor includes at least one of a first factor, a second factor, or a third factor; and the determining the target processing parameter based on the phantom image and the reference standard includes:
calculating, by a second preset manner, an expected value of a standard parameter based on an upper bound and a lower bound of the standard parameter in the EARL V2.0 standard, wherein the standard parameter in the EARL V2.0 standard includes at least one of a maximum recovery coefficient, a mean recovery coefficient, and a regional mean value of a peak recovery coefficient;
performing the first processing iteratively on the phantom image; and
determining at least one of the first factor, the second factor, or the third factor based on the expected value of the standard parameter.

8. The method of claim 7, wherein the second preset manner includes:
determining the expected value of the standard parameter based on an average value of the upper bound and the lower bound of the standard parameter.

9. The method of claim 1, wherein the determining an image processing result by processing an image to be processed based on the target processing parameter includes:
determining a normalized image by performing the second processing on the image to be processed based on the target processing parameter; and
determining the image processing result based on the normalized image, wherein the image processing result includes a normalized quantitative result.

10. A PET image processing system, comprising:
a first acquisition module (210),
configured to obtain a phantom image and a normalization standard, wherein the normalization standard includes a reference standard;
a first processing module, configured to perform a first processing on the phantom image, generate a first recovery coefficient of the processed phantom image generated by the first processing, and determine whether the first recovery coefficient satisfies the normalization standard;
a second acquisition module, configured to obtain a target processing parameter based on the phantom image and the reference standard in response to determining that the first recovery coefficient satisfies the normalization standard, based on the phantom image and the reference standard, wherein the target processing parameter is a normalization factor; and
a second processing module, configured to determine an image processing result by processing an image to be processed based on the target processing parameter;
wherein the image processing result includes a normalized standard uptake value; and the second processing module is further configured to:
perform a second processing on the image to be processed using the normalization factor to determine the normalized standard uptake value.

11. The system of claim 10, wherein the normalization standard further includes a customized standard; the image processing result includes a normalized standard uptake value; and the second processing module is further configured to:
in response to determining that the first recovery coefficient does not satisfy the reference standard, determine the customized standard as a target normalization standard; wherein the customized standard is determined by obtaining a user input parameter, and the user input parameter is determined based on a value of the first recovery coefficient corresponding to a sphere in a phantom;
obtain a second recovery coefficient by performing the first processing iteratively on the phantom image;
obtain the target processing parameter in response to determining that the second recovery coefficient satisfies the customized standard, wherein the target processing parameter is a processing parameter when the second recovery coefficient satisfies the customized standard;
perform the second processing on the image to be processed based on the target processing parameter; and
determine the normalized standard uptake value.

12. The system of claim 11, the customized standard including a maximum recovery coefficient, a mean recovery coefficient, and a regional mean value of a peak recovery coefficient, wherein each of the maximum recovery coefficient, the mean recovery coefficient, and the regional mean value of the peak recovery coefficient includes an expected value, an upper bound, and a lower bound, respectively; and the customized standard is determined by:
determining the expected value based on the value of the first recovery coefficient corresponding to the sphere; and
determining the upper bound and the lower bound based on the expected value.

13. The system of claim 11, wherein the reference standard includes at least one of an EARL V1.0 standard and an EARL V2.0 standard.

14. The system of claim 11, wherein the phantom image and the image to be processed are obtained based on a same acquisition condition and a same reconstruction condition.

15. A non-transitory computer-readable storage medium storing computer instructions, wherein when a computer reads the computer instructions in the storage medium, the computer performs the method of any one of the claims 1-9.

## Patentansprüche

1. PET-Bildverarbeitungsverfahren, umfassend:
Erhalten (310) eines Phantombildes und eines Normalisierungsstandards, wobei der Normalisierungsstandard einen Referenzstandard beinhaltet;
Durchführen einer ersten Verarbeitung des Phantombildes;
Erzeugen eines ersten Wiederherstellungskoeffizienten eines verarbeiteten Phantombildes, welcher durch Durchführen der ersten Verarbeitung des Phantombildes erzeugt wurde;
Bestimmen, ob der erste Wiederherstellungskoeffizient den Normalisierungsstandard erfüllt;
als Reaktion auf Bestimmen, dass der erste Wiederherstellungskoeffizient den Normalisierungsstandard erfüllt, Erhalten eines Zielverarbeitungsparameters basierend auf dem Phantombild und dem Referenzstandard, wobei der Zielverarbeitungsparameter ein Normalisierungsfaktor ist; und
Bestimmen des Bildverarbeitungsergebnisses durch Verarbeiten eines zu verarbeitenden Bildes basierend auf dem Zielverarbeitungsparameter;
wobei das Bildverarbeitungsergebnis einen normalisierten Standardaufnahmewert beinhaltet; und das Bestimmen eines Bildverarbeitungsergebnisses durch Verarbeiten eines zu verarbeitenden Bildes basierend auf dem Zielverarbeitungsparameter beinhaltet:
Durchführen einer zweiten Verarbeitung des zu verarbeitenden Bildes unter Verwendung des Normalisierungsfaktors, um den normalisierten Standardaufnahmewert zu bestimmen.

2. Verfahren nach Anspruch 1, wobei der Normalisierungsstandard weiter einen kundenspezifischen Standard beinhaltet; das Bildverarbeitungsergebnis einen normalisierten Standardaufnahmewert beinhaltet; und das Bestimmen eines Bildverarbeitungsergebnisses durch Verarbeiten eines zu verarbeitenden Bildes basierend auf dem Zielverarbeitungsparameter beinhaltet:
als Reaktion auf Bestimmen, dass der erste Wiederherstellungskoeffizient den Referenzstandard nicht erfüllt, Bestimmen des kundenspezifischen Standards als Zielnormalisierungsstandard; wobei der kundenspezifische Standard durch Erhalten eines Benutzereingabeparameters bestimmt wird, und der Benutzereingabeparameter basierend auf einem Wert des ersten Wiederherstellungskoeffizienten bestimmt wird, welcher einer Kugel in einem Phantom entspricht;
Erhalten eines zweiten Wiederherstellungskoeffizienten durch iteratives Durchführen der ersten Verarbeitung auf das Phantombild;
Erhalten des Zielverarbeitungsparameters als Reaktion auf Bestimmen, dass der zweite Wiederherstellungskoeffizient den kundenspezifischen Standard erfüllt, wobei der Zielverarbeitungsparameter ein Verarbeitungsparameter ist, wenn der zweite Wiederherstellungskoeffizient den kundenspezifischen Standard erfüllt;
Durchführen der zweiten Verarbeitung des zu verarbeitenden Bildes basierend auf dem Zielverarbeitungsparameter; und
Bestimmen des normalisierten Standardaufnahmewertes.

3. Verfahren nach Anspruch 2, wobei der kundenspezifische Standard einen maximalen Wiederherstellungskoeffizienten, einen mittleren Wiederherstellungskoeffizienten und einen regionalen Mittelwert eines Spitzenwiederherstellungskoeffizienten beinhaltet, wobei jeder von dem maximalen Wiederherstellungskoeffizienten, dem mittleren Wiederherstellungskoeffizienten und dem regionalen Mittelwert des Spitzenwiederherstellungskoeffizienten jeweils einen erwarteten Wert, eine obere Grenze und eine untere Grenze beinhaltet; und der kundenspezifische Standard bestimmt wird durch:
Bestimmen des erwarteten Wertes basierend auf dem Wert des ersten Wiederherstellungskoeffizienten, welcher der Kugel entspricht; und
Bestimmen der oberen Grenze und der unteren Grenze basierend auf dem erwarteten Wert.

4. Verfahren nach Anspruch 2, wobei der Referenzstandard zumindest einen von einem EARL V1.0-Standard und/oder einem EARL V2.0-Standard beinhaltet.

5. Verfahren nach Anspruch 2, wobei das Phantombild und das zu verarbeitende Bild basierend auf derselben Erfassungsbedingung und derselben Rekonstruktionsbedingung erhalten werden.

6. Verfahren nach Anspruch 1, wobei der Referenzstandard einen EARL V1.0-Standard beinhaltet, der Normalisierungsfaktor zumindest einen von einem ersten Faktor, einem zweiten Faktor oder einem dritten Faktor beinhaltet; und das Bestimmen des Zielverarbeitungsparameters basierend auf dem Phantombild und dem Referenzstandards beinhaltet:
iteratives Durchführen der ersten Verarbeitung des Phantombildes;
Bestimmen des ersten Faktors und des zweiten Faktors basierend auf einem erwarteten Wert eines Standardparameters in dem EARL V1.0-Standard, wobei der Standardparameter in dem EARL V1.0-Standard einen maximalen Wiederherstellungsfaktor und einen durchschnittlichen Wiederherstellungsfaktor beinhaltet; und
Bestimmen des dritten Faktors durch eine erste voreingestellte Art basierend auf dem ersten Faktor und dem zweiten Faktor, und wobei die erste voreingestellte Art Bestimmen des dritten Faktors basierend auf einem maximalen Wert des ersten Faktors und des zweiten Faktors beinhaltet.

7. Verfahren nach Anspruch 1, wobei der Referenzstandard einen EARL V2.0-Standard beinhaltet, der Normalisierungsfaktor zumindest einen von einem ersten Faktor, einem zweiten Faktor oder einem dritten Faktor beinhaltet; und das Bestimmen des Zielverarbeitungsparameters basierend auf dem Phantombild und dem Referenzstandard beinhaltet:
Berechnen eines erwarteten Wertes eines Standardparameters basierend auf einer oberen Grenze und einer unteren Grenze des Standardparameters in dem EARL V2.0-Standard durch eine zweite voreingestellte Art, wobei der Standardparameter in dem EARL V2.0-Standard zumindest eines von einem maximalen Wiederherstellungskoeffizienten, einem mittleren Wiederherstellungskoeffizienten und einem regionalen Mittelwert eines Spitzenwiederherstellungskoeffizienten beinhaltet;
iteratives Durchführen der ersten Verarbeitung des Phantombildes; und
Bestimmen zumindest eines von dem ersten Faktor, dem zweiten Faktor oder dem dritten Faktor basierend auf dem erwarteten Wert des Standardparameters.

8. Verfahren nach Anspruch 7, wobei die zweite voreingestellte Art beinhaltet:
Bestimmen des erwarteten Wertes des Standardparameters basierend auf einem Durchschnittswert der oberen Grenze und der unteren Grenze des Standardparameters.

9. Verfahren nach Anspruch 1, wobei Bestimmen des Bildverarbeitungsergebnisses durch Verarbeiten eines zu verarbeitenden Bildes basierend auf dem Zielverarbeitungsparameter beinhaltet:
Bestimmen eines normalisierten Bildes durch Durchführen der zweiten Verarbeitung des zu verarbeitenden Bildes basierend auf dem Zielverarbeitungsparameter; und
Bestimmen des Bildverarbeitungsergebnisses basierend auf dem normalisierten Bild, wobei das Bildverarbeitungsergebnis ein normalisiertes quantitatives Ergebnis beinhaltet.

10. PET-Bildverarbeitungssystem, umfassend:
ein erstes Erfassungsmodul (210), welches dazu konfiguriert ist, ein Phantombild und einen Normalisierungsstandard zu erhalten, wobei der Normalisierungsstandard einen Referenzstandard beinhaltet;
ein erstes Verarbeitungsmodul, welches dazu konfiguriert ist, eine erste Verarbeitung des Phantombildes durchzuführen, einen ersten Wiederherstellungskoeffizienten des verarbeiteten Phantombildes zu erzeugen, welcher durch die erste Verarbeitung erzeugt wurde, und zu bestimmen, ob der erste Wiederherstellungskoeffizient den Normalisierungsstandard erfüllt;
ein zweites Erfassungsmodul, welches dazu konfiguriert ist, basierend auf dem Phantombild und dem Referenzstandard als Reaktion auf Bestimmen, dass der erste Wiederherstellungskoeffizient den Normalisierungsstandard erfüllt, einen Zielverarbeitungsparameter basierend auf dem Phantombild und dem Referenzstandard zu erhalten, wobei der Zielverarbeitungsparameter ein Normalisierungsfaktor ist; und
ein zweites Verarbeitungsmodul, welches dazu konfiguriert ist, ein Bildverarbeitungsergebnis durch Verarbeiten eines zu verarbeitenden Bildes basierend auf dem Zielverarbeitungsparameter zu bestimmen;
wobei das Bildverarbeitungsergebnis einen normalisierten Standardaufnahmewert beinhaltet; und das zweite Verarbeitungsmodul weiter dazu konfiguriert ist:
eine zweite Verarbeitung des zu verarbeitenden Bildes unter Verwendung des Normalisierungsfaktors durchzuführen, um den normalisierten Standardaufnahmewert zu bestimmen.

11. System nach Anspruch 10, wobei der Normalisierungsstandard weiter einen kundenspezifischen Standard beinhaltet; das Bildverarbeitungsergebnis einen normalisierten Standardaufnahmewert beinhaltet; und das zweite Verarbeitungsmodul weiter dazu konfiguriert ist:
als Reaktion auf Bestimmen, dass der erste Wiederherstellungskoeffizient den Referenzstandard nicht erfüllt, den kundenspezifischen Standard als Zielnormalisierungsstandard zu bestimmen; wobei der kundenspezifische Standard durch Erhalten eines Benutzereingabeparameters bestimmt wird, und der Benutzereingabeparameter basierend auf einem Wert des ersten Wiederherstellungskoeffizienten bestimmt wird, welcher einer Kugel in einem Phantom entspricht;
einen zweiten Wiederherstellungskoeffizienten durch iteratives Durchführen der ersten Verarbeitung auf das Phantombild zu erhalten;
als Reaktion auf Bestimmen, dass der zweite Wiederherstellungskoeffizient den kundenspezifischen Standard erfüllt, den Zielverarbeitungsparameter zu erhalten, wobei der Zielverarbeitungsparameter ein Verarbeitungsparameter ist, wenn der zweite Wiederherstellungskoeffizient den kundenspezifischen Standard erfüllt;
die zweiten Verarbeitung des zu verarbeitenden Bildes basierend auf dem Zielverarbeitungsparameter durchzuführen; und
den normalisierten Standardaufnahmewert zu bestimmen.

12. System nach Anspruch 11, wobei der kundenspezifische Standard einen maximalen Wiederherstellungskoeffizienten, einen mittleren Wiederherstellungskoeffizienten und einen regionalen Mittelwert eines Spitzenwiederherstellungskoeffizienten beinhaltet, wobei jeder von dem maximalen Wiederherstellungskoeffizienten, dem mittleren Wiederherstellungskoeffizienten und dem regionalen Mittelwert des Spitzenwiederherstellungskoeffizienten jeweils einen erwarteten Wert, eine obere Grenze und eine untere Grenze beinhaltet; und der kundenspezifische Standard bestimmt wird durch:
Bestimmen des erwarteten Wertes basierend auf dem Wert des ersten Wiederherstellungskoeffizienten, welcher der Kugel entspricht; und
Bestimmen der oberen Grenze und der unteren Grenze basierend auf dem erwarteten Wert.

13. System nach Anspruch 11, wobei der Referenzstandard zumindest einen von einem EARL V1.0-Standard und einem EARL V2.0-Standard beinhaltet.

14. System nach Anspruch 11, wobei das Phantombild und das zu verarbeitende Bild basierend auf derselben Erfassungsbedingung und derselben Rekonstruktionsbedingung erhalten werden.

15. Nichtflüchtiges, computerlesbares Speichermedium, welches Computeranweisungen speichert, wobei, wenn ein Computer die Computeranweisungen in dem Speichermedium liest, der Computer das Verfahren nach einem der Ansprüche 1-9 durchführt.

## Revendications

1. Procédé de traitement d'images TEP, comprenant :
l'obtention (310) d'une image fantôme et d'une norme de normalisation, dans lequel la norme de normalisation inclut une norme de référence ;
la réalisation d'un premier traitement sur l'image fantôme ;
la génération d'un premier coefficient de récupération d'une image fantôme traitée, générée par réalisation du premier traitement sur l'image fantôme ;
la détermination si le premier coefficient de récupération est conforme à la norme de normalisation ;
en réponse à la détermination que le premier coefficient de récupération est conforme à la norme de normalisation, l'obtention d'un paramètre de traitement cible sur la base de l'image fantôme et la norme de référence, dans lequel le paramètre de traitement cible est un facteur de normalisation ; et
la détermination d'un résultat de traitement d'image par traitement d'une image à traiter sur la base du paramètre de traitement cible ;
dans lequel le résultat de traitement d'image inclut une valeur de fixation de norme normalisée ; et la détermination d'un résultat de traitement d'image par traitement d'une image à traiter sur la base du paramètre de traitement cible inclut :
la réalisation d'un second traitement sur l'image à traiter à l'aide du facteur de normalisation pour déterminer la valeur de fixation de norme normalisée.

2. Procédé selon la revendication 1, dans lequel la norme de normalisation inclut en outre une norme personnalisée ; le résultat de traitement d'image inclut une valeur de fixation de norme normalisée ; et la détermination d'un résultat de traitement d'image par traitement d'une image à traiter sur la base du paramètre de traitement cible inclut :
en réponse à la détermination que le premier coefficient de récupération n'est pas conforme à la norme de référence, la détermination de la norme personnalisée comme norme de normalisation cible ; dans lequel la norme personnalisée est déterminée par obtention d'un paramètre d'entrée utilisateur, et le paramètre d'entrée utilisateur est déterminé sur la base d'une valeur du premier coefficient de récupération correspondant à une sphère dans une image fantôme ;
l'obtention d'un deuxième coefficient de récupération par réalisation du premier traitement de manière itérative sur l'image fantôme ;
l'obtention du paramètre de traitement cible en réponse à la détermination que le deuxième coefficient de récupération est conforme à la norme personnalisée, dans lequel le paramètre de traitement cible est un paramètre de traitement lorsque le deuxième coefficient de récupération est conforme à la norme personnalisée ;
la réalisation du second traitement sur l'image à traiter sur la base du paramètre de traitement cible ; et
la détermination de la valeur de fixation de norme normalisée.

3. Procédé selon la revendication 2, la norme personnalisée incluant un coefficient de récupération maximal, un coefficient de récupération moyen et une valeur moyenne régionale d'un coefficient de récupération de pointe, dans lequel chacun du coefficient de récupération maximal, du coefficient de récupération moyen et de la valeur moyenne régionale du coefficient de récupération de pointe inclut respectivement une valeur attendue, une limite supérieure et une limite inférieure ; et la norme personnalisée est déterminée par :
la détermination de la valeur attendue sur la base de la valeur du premier coefficient de récupération correspondant à la sphère ; et
la détermination de la limite supérieure et de la limite inférieure sur la base de la valeur attendue.

4. Procédé selon la revendication 2, dans lequel la norme de référence inclut au moins une parmi une norme EARL V1.0 et une norme EARL V2.0.

5. Procédé selon la revendication 2, dans lequel l'image fantôme et l'image à traiter sont obtenues sur la base d'une même condition d'acquisition et d'une même condition de reconstruction.

6. Procédé selon la revendication 1, dans lequel la norme de référence inclut une norme EARL V1.0, le facteur de normalisation inclut au moins un parmi un premier, un deuxième et un troisième facteur ; et la détermination du paramètre de traitement cible sur la base de l'image fantôme et de la norme de référence inclut :
la réalisation du premier traitement de manière itérative sur l'image fantôme ;
la détermination du premier facteur et du deuxième facteur sur la base d'une valeur attendue d'un paramètre de norme dans la norme EARL V1.0, dans lequel le paramètre de norme dans la norme EARL V1.0 inclut un facteur de récupération maximal et un facteur de récupération moyen ;
la détermination du troisième facteur d'une première manière prédéfinie sur la base du premier facteur et du deuxième facteur, et la première manière prédéfinie inclut la détermination du troisième facteur sur la base d'une valeur maximale du premier facteur et du deuxième facteur.

7. Procédé selon la revendication 1, dans lequel la norme de référence inclut une norme EARL V2.0, le facteur de normalisation inclut au moins un parmi un premier facteur, un deuxième facteur et un troisième facteur ; et la détermination du paramètre de traitement cible sur la base de l'image fantôme et de la norme de référence inclut :
le calcul, d'une seconde manière prédéfinie, d'une valeur attendue d'un paramètre de norme sur la base d'une limite supérieure et d'une limite inférieure du paramètre de norme dans la norme EARL V2.0, dans lequel le paramètre de norme dans la norme EARL V2.0 inclut au moins un parmi un coefficient de récupération maximal, un coefficient de récupération moyen et une valeur moyenne régionale d'un coefficient de récupération de pointe ;
la réalisation du premier traitement de manière itérative sur l'image fantôme ; et
la détermination d'au moins un parmi le premier facteur, le deuxième facteur et le troisième facteur sur la base de la valeur attendue du paramètre de norme.

8. Procédé selon la revendication 7, dans lequel la seconde manière prédéfinie inclut :
la détermination de la valeur attendue du paramètre de norme sur la base d'une valeur moyenne de la limite supérieure et de la limite inférieure du paramètre de norme.

9. Procédé selon la revendication 1, dans lequel la détermination d'un résultat de traitement d'image par traitement d'une image à traiter sur la base du paramètre de traitement cible inclut :
la détermination d'une image normalisée par réalisation du second traitement sur l'image à traiter sur la base du paramètre de traitement cible ; et
la détermination du résultat de traitement d'image sur la base de l'image normalisée, dans lequel le résultat de traitement d'image inclut un résultat quantitatif normalisé.

10. Système de traitement d'images TEP, comprenant :
un premier module d'acquisition (210), configuré pour obtenir une image fantôme et une norme de normalisation, dans lequel la norme de normalisation inclut une norme de référence ;
un premier module de traitement, configuré pour réaliser un premier traitement sur l'image fantôme, générer un premier coefficient de récupération de l'image fantôme traitée générée par le premier traitement, et déterminer si le premier coefficient de récupération est conforme à la norme de normalisation ;
un second module d'acquisition, configuré pour obtenir un paramètre de traitement cible sur la base de l'image fantôme et de la norme de référence, en réponse à la détermination que le premier coefficient de récupération est conforme à la norme de normalisation, sur la base de l'image fantôme et de la norme de référence, dans lequel le paramètre de traitement cible est un facteur de normalisation ; et
un second module de traitement, configuré pour déterminer un résultat de traitement d'image par traitement d'une image à traiter sur la base du paramètre de traitement cible ;
dans lequel le résultat de traitement d'image inclut une valeur de fixation de norme normalisée ; et le second module de traitement est en outre configuré pour :
réaliser un second traitement sur l'image à traiter à l'aide du facteur de normalisation pour déterminer la valeur de fixation de norme normalisée.

11. Système selon la revendication 10, dans lequel la norme de normalisation inclut en outre une norme personnalisée ; le résultat de traitement d'image inclut une valeur de fixation de norme normalisée ; et le second module de traitement est en outre configuré pour :
en réponse à la détermination que le premier coefficient de récupération n'est pas conforme à la norme de référence, déterminer la norme personnalisée comme norme de normalisation cible ; dans lequel la norme personnalisée est déterminée par obtention d'un paramètre d'entrée utilisateur, et le paramètre d'entrée utilisateur est déterminé sur la base d'une valeur du premier coefficient de récupération correspondant à une sphère dans une image fantôme ;
obtenir un deuxième coefficient de récupération par réalisation du premier traitement de manière itérative sur l'image fantôme ;
obtenir le paramètre de traitement cible en réponse à la détermination que le deuxième coefficient de récupération est conforme à la norme personnalisée, dans lequel le paramètre de traitement cible est un paramètre de traitement lorsque le deuxième coefficient de récupération est conforme à la norme personnalisée ;
réaliser le second traitement sur l'image à traiter sur la base du paramètre de traitement cible ; et
déterminer la valeur de fixation de norme normalisée.

12. Système selon la revendication 11, la norme personnalisée incluant un coefficient de récupération maximal, un coefficient de récupération moyen et une valeur moyenne régionale d'un coefficient de récupération de pointe, dans lequel chacun du coefficient de récupération maximal, du coefficient de récupération moyen et de la valeur moyenne régionale du coefficient de récupération de pointe inclut respectivement une valeur attendue, une limite supérieure et une limite inférieure ; et la norme personnalisée est déterminée par :
la détermination de la valeur attendue sur la base de la valeur du premier coefficient de récupération correspondant à la sphère ; et
la détermination de la limite supérieure et de la limite inférieure sur la base de la valeur attendue.

13. Système selon la revendication 11, dans lequel la norme de référence inclut au moins une parmi une norme EARL V1.0 et une norme EARL V2.0.

14. Système selon la revendication 11, dans lequel l'image fantôme et l'image à traiter sont obtenues sur la base d'une même condition d'acquisition et d'une même condition de reconstruction.

15. Support de stockage non transitoire lisible par ordinateur stockant des instructions informatiques, dans lequel, lorsqu'un ordinateur lit les instructions informatiques dans le support de stockage, l'ordinateur réalise le procédé selon l'une quelconque des revendications 1-9.
